# EUROPEAN PATENT SPECIFICATION

(11) **EP 3 886 902 B1**
(45) Date of publication and mention of the grant of the patent: **18.11.2026**
(21) Application number: 19816253.9
(22) Date of filing: 02.12.2019
(51) Int. Cl.: C07K 16/08, A61K 9/00, A61K 35/16, A61K 39/395, A61P 11/00, A61P 31/00, C07K 16/12, A61K 39/00

(54) **COMPOSITION COMPRISING HUMAN PLASMA-DERIVED IGG FOR USE IN THE PREVENTION OR TREATMENT OF AN ACUTE EXACERBATION IN A HUMAN SUBJECT WITH CHRONIC OBSTRUCTIVE PULMONARY DISEASE (COPD) OR NON-CYSTIC FIBROSIS BRONCHIECTASIS (NCFB)**
ZUSAMMENSETZUNG, DIE AUS MENSCHLICHEM PLASMA GEWONNENES IGG ENTHÄLT, ZUR ANWENDUNG BEI DER VORBEUGUNG ODER BEHANDLUNG EINER AKUTEN EXAZERBATION BEI EINEM MENSCHLICHEN PATIENTEN MIT CHRONISCHER OBSTRUKTIVER LUNGENERKRANKUNG (COPD) ODER BRONCHIEKTASIE OHNE ZYSTISCHE FIBROSE (NCFB)
COMPOSITION CONTENANT DES IGG DÉRIVÉES DU PLASMA HUMAIN, POUR SA UTILISATION DANS LA PRÉVENTION OU LE TRAITEMENT D'UNE EXACERBATION AIGUË CHEZ UN SUJET HUMAIN ATTEINT D'UNE MALADIE PULMONAIRE OBSTRUCTIVE CHRONIQUE (MPOC) OU DE BRONCHECTASIES NON LIÉES À LA FIBROSE KYSTIQUE (NCFB)

(30) Priority: 30.11.2018 EP 18209556
(43) Date of publication of application: 06.10.2021
(60) Divisional application: 26212234.4
(73) Proprietor: CSL Behring AG, 3014 Bern (CH)
(72) Inventor: VONARBURG, Cédric Pierre, 3074 Muri bei Bern (CH); SCHULZE, Ilka, 3095 Spiegel b. Bern (CH)
(74) Representative: CSL Global IP
(86) International application number: PCT/EP2019/083271
(87) International publication number: WO 2020/109621

(56) References cited:
- US-A1- 2008 145 371
- US-A1- 2010 260 853
- US-A1- 2017 021 114
- US-B2- 6 932 967
- WEDZICHA ET AL: "COPD exacerbations: defining their cause and prevention", THE LANCET, ELSEVIER, AMSTERDAM, NL, vol. 370, no. 9589, 1 September 2007 (2007-09-01), pages 786 - 796, XP022223439, ISSN: 0140-6736, DOI: 10.1016/S0140-6736(07)61382-8
- "Bronchiectasis : The EMBARC Manual", 1 January 2018, SPRINGER INTERNATIONAL PUBLISHING, Cham, ISBN: 978-3-319-61452-6, article POLVERINO EVA ET AL: "Exacerbation of Bronchiectasis : The EMBARC Manual", pages: 205 - 222, XP093123552, DOI: 10.1007/978-3-319-61452-6_15
- LOMMATZSCH MAREK ET AL: "IgE is associated with exacerbations and lung function decline in COPD", vol. 23, no. 1, 4 January 2022 (2022-01-04), GB, XP093123587, ISSN: 1465-993X, Retrieved from the Internet <URL:https://link.springer.com/article/10.1186/s12931-021-01847-0/fulltext.html> DOI: 10.1186/s12931-021-01847-0
- DANI BHAS ET AL: "High Concentration Formulation Feasibility of Human Immunoglubulin G for Subcutaneous Administration", JOURNAL OF PHARMACEUTICAL SCIENCES, vol. 96, no. 6, 1 June 2007 (2007-06-01), US, pages 1504 - 1517, XP093123589, ISSN: 0022-3549, DOI: 10.1002/jps.20508
- COWAN JUTHAPORN ET AL: "A Retrospective Longitudinal Within-Subject Risk Interval Analysis of Immunoglobulin Treatment for Recurrent Acute Exacerbation of Chronic Obstructive Pulmonary Disease", PLOS ONE, vol. 10, no. 11, 11 November 2015 (2015-11-11), US, pages e0142205, XP093330978, ISSN: 1932-6203, DOI: 10.1371/journal.pone.0142205
- MCCULLAGH BRIAN N. ET AL: "Antibody deficiency in patients with frequent exacerbations of Chronic Obstructive Pulmonary Disease (COPD)", PLOS ONE, vol. 12, no. 2, 17 February 2017 (2017-02-17), US, pages e0172437, XP093330980, ISSN: 1932-6203, DOI: 10.1371/journal.pone.0172437
- WYE VAN J E ET AL: "PSEUDOMONAS HYPERIMMUNE GLOBULIN PASSIVE IMMUNOTHERAPY FOR PULMONARY EXACERBATIONS IN CYSTIC FIBROSIS", PEDIATRIC PULMONOLOGY, JOHN WILEY, NEW YORK, NY, US, vol. 9, no. 1, 1 January 1990 (1990-01-01), pages 7 - 18, XP008031011, ISSN: 8755-6863
- PECORARO ANTONIO ET AL: "Immunoglobulin replacement therapy in primary and secondary antibody deficiency: The correct clinical approach", INTERNATIONAL IMMUNOPHARMACOLOGY, ELSEVIER, AMSTERDAM, NL, vol. 52, 12 October 2017 (2017-10-12), pages 136 - 142, XP085214622, ISSN: 1567-5769, DOI: 10.1016/J.INTIMP.2017.09.005
- WELTE TOBIAS ET AL: "Efficacy and safety of trimodulin, a novel polyclonal antibody preparation, in patients with severe community-acquired pneumonia: a randomized, placebo-controlled, double-blind, multicenter, phase II trial (CIGMA study)", INTENSIVE CARE MEDICINE, BERLIN, DE, vol. 44, no. 4, 9 April 2018 (2018-04-09), pages 438 - 448, XP036490175, ISSN: 0342-4642, [retrieved on 20180409], DOI: 10.1007/S00134-018-5143-7

## Description

### TECHNICAL FIELD

This invention is in the field of preventing or treating acute exacerbations in chronic lung diseases, such as chronic obstructive pulmonary disease and non-cystic fibrosis bronchiectasis, by administration of polyclonal immunoglobulin to the respiratory tract, in particular by direct application of an aerosolized composition comprising human plasma-derived IgG.

### BACKGROUND

Chronic lung diseases, in particular those that involve exacerbations where infections are the main driver, are characterized by difficulty for a subject to exhale the air in their lungs fully. Patients with such a chronic lung disease have shortness of breath due to difficulty exhaling all the air from the lungs. Because of damage to the lungs or narrowing of the airways inside the lungs, exhaled air comes out more slowly than normal. At the end of a full exhalation, an abnormally high amount of air may still linger in the lungs. Chronic obstructive pulmonary disease (COPD) and non-cystic fibrosis bronchiectasis (NCFB) are examples of such chronic lung diseases. COPD is characterized by persistent airflow limitation that is usually progressive and associated with an enhanced chronic inflammatory response in the airways and the lung to noxious particles or gases. Exacerbations and comorbidities contribute to the overall severity in individual patients [1]. NCFB is characterized by pathological dilation of the airways - clinically identified by radiographic demonstration of airway enlargement (i.e. by a CT scan) [2]. Exacerbations are considered to be key events in the progression of NCFB [3].

Acute exacerbations of respiratory symptoms often occur in patients with chronic lung diseases, such as COPD and NCFB. These acute exacerbations may be triggered by infection with bacteria or viruses (which may coexist). During exacerbations, there is a flare-up of inflammation, increased hyperinflation and gas trapping, reduced expiratory flow, and increased dyspnea. Other medical conditions, such as pneumonia may aggravate an exacerbation of e.g. COPD.

An acute exacerbation of COPD is defined by the global initiative for chronic obstructive lung disease (GOLD) as an acute worsening of the patient's respiratory symptoms that is beyond normal day-to-day variations that results in additional therapy medication [4]. The rate at which exacerbations occur varies greatly between patients. Chronicity of exacerbations in patients with COPD support tissue remodeling of the airways and contribute to the aggravation of the disease. These acute exacerbations correlate with a high degree of systemic inflammation and immune activation. As COPD severity worsens, the frequency of exacerbations increases. In turn, acute exacerbations likely increase the progression of COPD, and additionally, it is likely that the inflammatory state created by the acute exacerbations increases susceptibility to additional, recurrent acute exacerbations. This leads to a vicious cycle driving progression of COPD.

An exacerbation of NCFB may be defined as the acute worsening of one or more symptoms of NCFB beyond normal day-to-day variations, for example the requirement of antibiotics in the presence of one or more symptoms such as increasing cough, increasing sputum volume, or worsening sputum purulence. A severe exacerbation may be defined as requiring unscheduled hospitalization or an emergency department visit [3].

Patients with chronic lung diseases, such as COPD or NCFB, are likely to present with recurrent respiratory tract infections, which can trigger an acute exacerbation. The most common causes of acute exacerbations of COPD are viral infections of the upper respiratory tract and the tracheobronchial tree. The most common viruses detected during COPD exacerbations are human rhinoviruses (HRV) [5], which are associated with an outgrowth of the bacterial airway microbiome [6]. Bacterial flora in COPD is generally highly variable. Many different bacteria have been associated with COPD. However, the most pathogenic ones include *Haemophilus influenza, Streptococcus pneumonia, Moraxella catarrhalis, Haemophilus parainfluenzae* and *Staphylococcus aureus.* In addition, *Pseudomonas aeruginosa* (PA) has been described to be one of the most harmful bacteria found in patients with excessively severe airflow obstruction in stable COPD and during exacerbations [7].

Treatments for COPD are based on inhaled corticosteroids (ICS), inhaled bronchodilators including long-acting beta2-agonists, and anticholinergics including long-acting muscarinic receptor antagonists, and combinations of these. For example, severe COPD with a high risk of exacerbations is commonly treated with a combination of all three classes of drugs. These therapies reduce exacerbations but patients taking maximum inhaled therapy continue to experience exacerbations and therefore new therapeutic approaches are needed. Indeed, ICS therapy is associated with side effects, including high risk of pneumonia, oral candidiasis, hoarse voice and skin bruising. Other side effects include increased risk of new-onset diabetes, diabetes progression, cataracts and tuberculosis. Long-term use is also associated with increased risk of bone fractures in COPD patients [8]. In particular, ICS therapy only modestly reduces the frequency of exacerbations and clinical trials report an increased risk of pneumonia with ICS use in COPD. This may be because ICSs appear to reduce antiviral immunity, leading to mucus hypersecretion and increased lung bacterial loads [9].

In COPD patients with chronic bronchitis, a phosphodiesterase-4 enzyme inhibitor (e.g. roflumilast) can be added to the selected treatment. Roflumilast is a non-steroid, anti-inflammatory active substance designed to target both the systemic and pulmonary inflammation associated with COPD. It is indicated for maintenance treatment of severe COPD associated with chronic bronchitis in adult patients with a history of frequent exacerbations as an adjunct therapy to bronchodilator treatment.

Acute exacerbations of COPD are currently managed with pharmacological therapies including bronchodilators, ICS and antibiotics. ICS therapy is associated with side effects, as discussed above. Antibiotics are used to treat bacterial respiratory tract infections, in order to reduce occurrence and severity of exacerbations. Macrolides also have an anti-inflammatory effect and may be used in patients with severe COPD and a history of frequent exacerbations. However, long-term macrolide therapy is associated with risk of microbial resistance and cardiovascular adverse effects. There are currently no agents for treating viral infections, such as rhinovirus infections, in COPD.

There are no treatments available for NCFB. Acute exacerbations of NCFB are commonly treated with antibiotics, to eliminate underlying respiratory tract infection. Some NCFB patients receive prophylactic antibiotic therapy to prevent exacerbations; however, the efficacy of such therapy has not been proven.

According to US 2017/021114 A1 a nebulizer device and a method to administer a polyclonal immunoglobulin to the respiratory tract of a subject is described. The use of a membrane nebulizer in a method for generating an aerosol of polyclonal immunoglobulins is demonstrated. However, said disclosure focuses on providing a method for generating an aerosol by use of a membrane nebulizer in general. Although the diseases COPD and NCFB are mentioned in a list of several indications, the reference is silent with regard to any prevention or treatment of an acute exacerbation in a human subject with said chronic lung diseases caused by viral or bacterial infection of the respiratory tract.

COWAN JUTHAPORN ET AL: "A Retrospective Longitudinal Within-Subject Risk Interval Analysis of Immunoglobulin Treatment for Recurrent Acute Exacerbation of Chronic Obstructive Pulmonary Disease",PLOS ONE, vol. 10, no. 11, 11 November 2015 (2015-11-11) reveals intravenous administration of a composition comprising polyvalent IgG for the prevention of acute exacerbations of COPD (AECOPD) from viral and/or bacterial infection. This study is silent on administration of IgG to the respiratory tract of the subject.

It is an object of the present disclosure to provide further and improved compositions for prevention or treatment of bacteria or virus induced acute exacerbations in the chronic lung diseases COPD and NCFB.

### DISCLOSURE OF THE INVENTION

In contrast to prior art therapies for preventing or treating acute exacerbations, which are based on antibiotics, optionally in combination with corticosteroids, beta2-agonists and/or anticholinergic bronchodilators, according to the invention as defined in claim 1 acute exacerbations are prevented or treated by administration of a composition comprising polyclonal immunoglobulin to the respiratory tract of a human subject.

The invention provides a composition for use according to claim 1. Thus, the invention provides a composition comprising human plasma-derived IgG for use in the prevention or treatment of an acute exacerbation in a human subject with a chronic lung disease, wherein the composition is administered to the respiratory tract of the subject, wherein the chronic lung disease is chronic obstructive pulmonary disease (COPD) or non-cystic fibrosis bronchiectasis (NCFB), and wherein the acute exacerbation is caused by a rhinovirus respiratory tract infection or a Pseudomonas aeruginosa infection of the respiratory tract. Further embodiments are described in the dependent claims.

Surprisingly, application of immunoglobulin to the mucosal epithelium of the respiratory tract may reduce inflammation, drive immune exclusion of potentially pathogenic microbes (e.g. bacteria and/or viruses) present in the mucosal layer, and prevent direct damage to the epithelium, for example by bacterial exoenzymes and toxins, and/or viral replication (shedding). These effects are advantageous for preventing or treating an acute exacerbation, which may be caused by respiratory tract infections with e.g. bacteria and/or viruses.

Commercially available immunoglobulin compositions are administered intravenously or subcutaneously, i.e. by systemic administration. Direct local administration to the target respiratory tract, e.g. by aerosol inhalation, may achieve the same exposure to immunoglobulin in the respiratory tract but require a smaller total dose than would be required for systemic administration (e.g. intravenous). This localized administration direct to the target respiratory tract tissue may thereby avoid systemic side effects. In addition, administration to the respiratory tract may enable a higher local concentration to be achieved than could be achieved by systemic administration, because only a proportion of systemically administered immunoglobulin ends up in the respiratory tract.

Furthermore, intravenous or subcutaneous immunoglobulin therapy is expensive. Targeted, localized administration direct to the respiratory tract may require the administration of a smaller dose to achieve the same exposure to immunoglobulin, e.g. IgG, in the respiratory tract as would be obtained by systemic administration. As a result, direct administration to the respiratory tract may be more cost-effective, because less composition is required to achieve the same therapeutic effect in the respiratory tract.

In addition, intravenous or subcutaneous immunoglobulin therapy usually requires the attention of a healthcare professional. For example, intravenous administration requires a nurse or physician and is usually performed in the clinic. Inhalation of an aerosol immunoglobulin may not require supervision by a healthcare profession and may therefore be suitable for self-administration at home. Consequently, direct administration to the respiratory tract may be more practical for the subject and therefore the subject may be more likely to comply with the treatment. Increased compliance reduces therapeutic failures, which can lead to e.g. acute exacerbations and hospitalization.

### Acute exacerbations

The invention involves the prevention or treatment of an acute exacerbation in a human subject with the chronic lung disease COPD or NCFB.

An acute exacerbation is an acute event characterized by a worsening of the patient respiratory symptoms that is beyond normal day-to-day variations and that requires additional therapy. Such acute exacerbations can be of different severity.

In a subject with COPD, a mild acute exacerbation is one that requires change of medication for the subject, in particular the subject is treated with a short-acting bronchodilator (SABD). A moderate acute exacerbation requires medical intervention, in particular treatment with an SABD plus an antibiotic and/or an oral corticosteroid. A severe acute exacerbation requires hospitalization or a visit to the emergency department. The subject of the invention may have a mild, moderate or severe acute exacerbation. Typically, the subject has a moderate or severe acute exacerbation.

In a subject with NCFB, an acute exacerbation is characterized by worsening local symptoms (cough, increased sputum volume or change of viscosity, increased sputum purulence with or without increasing wheeze, breathlessness, haemoptysis) and/or systemic upset [10]. A severe acute exacerbation may be characterized as one requiring unscheduled hospitalization or a visit to the emergency department [3].

The composition of the invention is for use in the prevention of an acute exacerbation, i.e. prophylactic therapy. In a specific embodiment, the composition of the invention is for use in the prevention of an acute exacerbation, wherein the composition prevents and/or treats an infection establishing in the respiratory tract of the subject. This prophylactic therapy may be particularly effective because it can prevent viral infections as well as bacterial infections and is effective against bacteria with resistance to one or more antibiotics.

Accordingly, the composition of the invention is for use in the prevention of an acute exacerbation, in particular by treating and/or preventing an underlying infection in the respiratory tract. Polyclonal immunoglobulin is particularly suitable because it can treat viral infections as well as bacterial infections, and is effective against bacteria with resistance to one or more antibiotics.

The composition of the invention is used in the treatment of an acute exacerbation. The acute exacerbation is caused by a viral or bacterial infection of the respiratory tract. The polyclonal immunoglobulin recognizes a broad spectrum of potentially pathogenic microbes (typically bacteria and viruses) in the respiratory tract. Recognizing a broad spectrum of bacteria means that the immunoglobulin is effective for treatment of a bacterial respiratory tract infection, for example by immune exclusion. Recognizing a broad spectrum of viruses means that the immunoglobulin is effective for treatment of a viral respiratory tract infection, for example by preventing viral binding to the host cell and thereby preventing viral replication and shedding. It may also be used effectively without the need for diagnostic tests to identify the specific bacterial or viral infection that may cause, or is causing, the acute exacerbation, which means that administration of the composition can be started sooner.

Treating an acute exacerbation may prevent the severity of the acute exacerbation worsening. For example, treating a mild acute exacerbation may prevent it progressing to a severe acute exacerbation.

The composition of the invention is for use in the treatment or prevention of an acute exacerbation. For this treatment or prevention of acute exacerbations, the composition of the invention may be enriched for an antibody that recognizes a specific pathogen. In one embodiment, the subject with an acute exacerbation is tested to identify the pathogen (e.g. bacteria and/or virus) causing the infection underlying the acute exacerbation and the composition of the invention is enriched with an antibody specific to the identified pathogen. In one embodiment, the composition of the invention is enriched by supplementing the composition with a monoclonal antibody specific to the identified pathogen. In another embodiment, the composition of the invention is enriched by supplementing with a monoclonal antibody specific to a pathogen that has been identified in the respiratory tract of the subject. In addition, or alternatively, the composition may be enriched with polyclonal immunoglobulin specific for certain pathogens, which can be obtained, for example, by immunization of a transgenic animal which has been engineered to produce human immunoglobulins, or by screening a library of the human antibody repertoire for antibodies with specificity for the desired pathogen(s), and then producing the identified antibodies recombinantly.

In a specific embodiment, the composition is for use in preventing or treating superinfection in the respiratory tract of the subject. A superinfection is a second infection that occurs in the respiratory tract during a first infection of the respiratory tract. In particular, a respiratory tract superinfection may occur with infection by a second infectious agent, which is resistant to the treatment being used against the first infectious agent. In one embodiment, both infections are bacterial respiratory tract infections. In another embodiment, the infections comprise one bacterial respiratory tract infection and one viral respiratory tract infection.

In one embodiment, the subject of the invention has a respiratory tract infection caused by bacteria, i.e. Pseudomonas aeruginosa, resistant to at least one antibiotic. In particular, the bacteria may be resistant to multiple antibiotics (multiply resistant). The composition of the invention is effective against these resistant bacteria, including multiply resistant bacteria, because the polyclonal immunoglobulin recognizes many epitopes on the bacteria, including epitopes unrelated to mechanisms of antibiotic activity and resistance.

In one embodiment, the subject of the invention has a respiratory tract infection caused by a virus, i.e. a rhinovirus. The composition of the invention recognizes the virus and treats the infection. In particular, the polyclonal immunoglobulin binds to the virus and prevents the virus binding to its host cell, e.g. an epithelial cell. The polyclonal immunoglobulin prevents viral entry into host cell, viral replication and viral shedding. This treatment may be particularly useful because there are no effective antiviral agents for use in treating respiratory tract infections.

### Patient history

The subject of the invention is a patient with a chronic lung disease that has a history of acute exacerbations.

The rate of acute exacerbations may vary between subjects. A subject of the invention may have frequent acute exacerbations, e.g. they experience two or more acute exacerbations per year. One of the best predictors of a subject having frequent acute exacerbations is a history of previous treated acute exacerbations. The composition of the invention is therefore particularly useful for treatment of a subject at risk of frequent acute exacerbations, which corresponds to a subject having a history of exacerbations. Therefore, in one embodiment, the composition of the invention is for use in the prevention or treatment of an acute exacerbation in a subject, wherein the subject has experienced one or more acute exacerbations in the 12 months prior to the prevention or treatment. Preferably, the subject has experienced two or more acute exacerbations in the 12 months prior to the prevention or treatment. In particular, the subject has experienced three or more acute exacerbations in the 12 months prior to the prevention or treatment.

Maintenance therapy (discussed below) is particularly suitable for such subjects with a history of acute exacerbations. Therefore, in a specific embodiment, the subject of the invention has experienced at least one acute exacerbation in the 12 months prior to the therapy and is treated with the composition for at least 12 months.

Specifically, in COPD, one of the strongest predictors of a patient's future acute exacerbation frequency is the number of acute exacerbations experienced in the previous year [4]. In particular, a subject with COPD that has experienced two or more acute exacerbations in the previous year is likely to have frequent exacerbations. Therefore, in a specific embodiment, the composition of the invention is for use in the prevention an acute exacerbation in a subject with COPD, wherein the prevention is maintenance therapy in a subject with COPD, and wherein the subject has experienced two or more acute exacerbations in the 12 months prior to the maintenance therapy starting and maintenance therapy continues for at least 12 months.

Specifically, in NCFB, one of the strongest predictors of a patient's future acute exacerbation frequency is the number of acute exacerbations experienced in the previous year [3]. In particular, a subject with NCFB that has experienced three or more acute exacerbations in the previous year is likely to have frequent exacerbations. Therefore, in a specific embodiment, the composition of the invention is for use in the prevention an acute exacerbation in a subject with NCFB, wherein the prevention is maintenance therapy in a subject with NCFB, and wherein the subject has experienced three or more acute exacerbations in the 12 months prior to the maintenance therapy starting and maintenance therapy continues for at least 12 months.

### Chronic lung disease

The invention involves the prevention or treatment of an acute exacerbation in a subject with the chronic lung disease COPD or NCFB, where infections are the main driver for exacerbations.

### COPD

The subject with COPD typically has a post-bronchodilator FEV1 (forced expiratory volume at 1s) / FVC (forced vital capacity) ratio of less than 0.7. FEV1 and FVC can be measured by spirometry, using standard methods in the art [11]. By way of example, for post-bronchodilator spirometry measurements, the spirometry may be performed: (i) 10-15 minutes after a short-acting beta2-agonist (400 µg) is administered; (ii) 30-45 minutes after a short-acting anticholinergic (160 µg) is administered; or (iii) 30-45 minutes after a combination of the two classes of drugs is administered.

The invention is particularly suitable for prevention or treatment of an acute exacerbation in a subject with moderate to very severe COPD, i.e. moderate COPD, severe COPD, or very severe COPD. Typically, the subject has severe or very severe COPD.

Such grading of the severity of COPD is explained in reference [1], and is based on the severity of airflow limitation in a subject. Briefly, in a subject with an FEV1/FVC ratio <0.7, the grading of severity of airflow limitation is based on the measured post-bronchodilator FEV1, and how this measured value compares to a predicted value for a healthy subject. A subject with mild COPD has an FEV1 at least 80% of predicted. A subject with moderate COPD has an FEV1 from 50% to 80% of predicted. A subject with severe COPD has an FEV1 from 30% to 50% of predicted. A subject with very severe COPD has an FEV1 less than 30% of predicted.

The predicted FEV1 for a healthy subject is calculated using the formula [12]: $Male FEV 1 \left\{litres\right\} = 4.30 * height \left\{metres\right\} - 0.029 * age \left\{years\right\} - 2.49$ $Female FEV 1 \left\{litres\right\} = 3.95 * height \left\{metres\right\} - 0.025 * age \left\{years\right\} - 2.60$

By way of example, a male subject aged 50 and 1.8m tall would have a predicted FEV1 of 3.8 L (4.3*1.8 - 0.029*50 - 2.49). If this subject was then measured by spirometry to have a post-bronchodilator FEV1 of 2.09 L, then this value would be 55% of the predicted FEV1 (3.8 L), and so the subject would be considered to have moderate COPD.

Moderate to very severe COPD is difficult to treat and even triple therapy (inhaled corticosteroid/beta2-agonist/anticholinergic bronchodilator) is not always successful. The polyclonal immunoglobulin of the present invention is thought to prevent or treat an acute exacerbation in a subject with COPD by mechanisms (including preventing respiratory tract infection and reducing respiratory tract inflammation) that are distinct from the mechanisms of current treatments and so provides a further and complementary treatment.

In another aspect, the composition of the invention is for use in treating COPD in a subject, wherein the composition is administered to the respiratory tract of the subject. Acute exacerbations contribute to the pathology of COPD and may contribute to the vicious cycle between inflammation and further infections. Preventing an acute exacerbation is therefore treatment of COPD. Maintenance therapy using the composition of the invention (discussed below) in a subject with COPD is particularly suitable for treating COPD, because it prevents an acute exacerbation (which encompasses reducing the incidence of acute exacerbations and/or reducing the severity). For similar reasons, seasonal administration of the composition of the invention is particularly suitable for treating COPD.

### Non-cystic fibrosis bronchiectasis

The invention involves the treatment of an acute exacerbation in a subject with a chronic lung disease, in particular the chronic lung disease NCFB where infections are the main driver for exacerbations. NCFB has multiple causes and can present with a broad array of signs. It is characterized by pathological dilation of the airways. In particular, it is defined as permanent enlargement of the airways [2], which can be demonstrated radiographically, e.g. by a computed tomography (CT) scan. Signs of NCFB span subtle dilation to cystic changes in the airways. Patients may be asymptomatic (and the airway dilation discovered unexpectedly), or may have a range of symptoms, such as cough and/or sputum production, with periodic exacerbations.

The invention is particularly suitable for prevention or treatment of an acute exacerbation in a subject with NCFB. In one embodiment, the composition of the invention is for use in preventing or treating an acute exacerbation in a subject with NCFB. The composition is particularly suitable for use in preventing a severe acute exacerbation in a subject with NCFB. In another aspect, the composition of the invention is for use in treating NCFB in a subject, wherein the composition is administered to the respiratory tract of the subject. Acute exacerbations contribute to the pathology of NCFB and may contribute to the vicious cycle between inflammation and further infections. Preventing an acute exacerbation is therefore treatment of NCFB. Maintenance therapy using the composition of the invention (discussed below) in a subject with NCFB is particularly suitable for treating NCFB, because it prevents an acute exacerbation (which encompasses reducing the incidence of acute exacerbations and/or reducing the severity). For similar reasons, seasonal administration of the composition of the invention is particularly suitable for treating NCFB.

A subject may present with COPD and NCFB as co-morbidities. Indeed, NCFB is associated with more advanced stages of COPD [2]. Therefore, in another embodiment, the composition of the invention is particularly suitable for use in prevention or treatment of an acute exacerbation in a subject with COPD and NCFB.

### Low IgG level

The subject of the invention may be one with a lower level of immunoglobulin G (IgG) than the normal range for a healthy adult. These subjects have an increased risk of suffering from COPD, an increased severity of COPD, and/or an increased risk of acute exacerbations of COPD. NCFB is also a common manifestation of subjects with immune deficiencies, including a low level of IgG.

IgG in the respiratory tract, in particular the lungs, comes from two sources: locally produced by plasma cells located in the bronchial mucosa, and derived from plasma by transudation. Accordingly, the subject of the invention may have a low level of IgG in the respiratory tract, e.g. because of a low systemic level of IgG and/or low local production of IgG.

In one embodiment, the subject has a low plasma level of IgG. As set out in reference [13], the normal range of total plasma IgG in healthy adults is 639-1,349 mg/dL, with a mean of 994 mg/dL. A low level of plasma IgG in an adult may be a level less than 700 mg/dL. A lower total plasma IgG level in an adult may be classified as mild-moderate (300-600 mg/dL), significant (100-300mg/dL), or profoundly reduced (less than about 100 mg/dL). In a particular embodiment, the subject has a plasma IgG level less than about 700 mg/dL, less than about 600 mg/dL, less than about 300 mg/dL, or less than about 100 mg/dL. In some embodiments, the subject has a plasma IgG level in the range of about 100 to about 600 mg/dL, (including in the ranges of about 300 to about 600 mg/dL, or about 100 to about 300 mg/dL).

Various methods for determining total plasma IgG concentration are known in the art, for example rate nephelometry and/or radial immunodiffusion [14]. Serum IgG can also be quantified by ELISA, e.g. according to the protocol described in the modes for carrying out the invention below.

The acute exacerbations that are prevented or treated by the invention manifest in the respiratory tract, and so the local concentration of IgG in the respiratory tract is an important factor in determining risk of respiratory tract infections and therefore also of acute exacerbations. A subject with a lower level of IgG in the respiratory tract than in a healthy adult is at greater risk of respiratory tract infection and an acute exacerbation. Therefore, in one embodiment, the subject of the invention has a lower level of IgG in the respiratory tract than a healthy adult.

The level of IgG in the respiratory tract can be measured by analyzing sputum from the subject. Sputum is a mixture of saliva and mucus coughed up from the respiratory tract, typically as a result of infection or other disease, such as COPD or NCFB. It is often examined microscopically to aid medical diagnosis. It can also be analysed for the content of biological molecules, including immunoglobulins (e.g. IgG, IgA and/or IgM), and cytokines (e.g. IL-1β, IL-6 and/or IL-8). Various methods for determining sputum immunoglobulin concentration are known in the art, for example rate nephelometry and/or radial immunodiffusion [15]. Sputum immunoglobulin can also be quantified by ELISA, e.g. according to the protocol described in the modes for carrying out the invention below.

### Therapeutic effects of the invention

### Prevention and/or treatment of respiratory tract infection

A respiratory tract infection may be one cause of an acute exacerbation, and so preventing or treating respiratory tract infection is particularly useful for the prevention or treatment of an acute exacerbation. In one embodiment, the composition of the invention is for use in the prevention of an acute exacerbation, wherein the human plasma-derived IgG causes immune exclusion of one or more potentially pathogenic microbes (e.g. bacteria and/or virus) in the respiratory tract. The polyclonal immunoglobulin may cause immune exclusion by binding to the potentially pathogenic microbes in the respiratory tract, for example, the polyclonal immunoglobulin binds to the potentially pathogenic microbes and prevents them adhering to the mucosal epithelium of the respiratory tract.

In another embodiment, the composition of the invention is for use in the prevention or treatment of an acute exacerbation, wherein the human plasma-derived IgG causes one or more potentially pathogenic microbes (e.g. bacteria and/or virus) in the respiratory tract to aggregate. Aggregation of the microbes is also known as agglutination.

In another embodiment, the composition of the invention is for use in the prevention or treatment of an acute exacerbation, wherein the human plasma-derived IgG recruits immune cells to kill the microbes, for example in a process termed antibody-dependent cellular cytotoxicity (ADCC).

### Prevention and/or reduction of damage caused by respiratory tract infection

The activity of microbes in the respiratory tract of a subject may have pathogenic effects. Therefore, in one embodiment, the composition of the invention is for use in the prevention or treatment of an acute exacerbation, wherein the human plasma-derived IgG reduces damage to the respiratory tract caused by pathogens (e.g. bacteria and/or virus). For example, the human plasma-derived IgG may inhibit the activity of exoenzymes. Such exoenzymes are enzymes secreted into the mucosa by e.g. bacteria, and include for example enzymes with tissue degrading activity, such as proteases. Blocking the activity of such exoenzymes protects the subject's respiratory tract epithelium from damage. In a specific embodiment, the composition of the invention prevents loss of epithelial barrier integrity and prevents passage of the pathogens across the epithelium. In a specific embodiment, the pathogen is a virus and the polyclonal immunoglobulin binds to the virus and prevents direct binding of the virus to a host cell in the respiratory tract of the subject. The immunoglobulin therefore prevents viral entry, replication and shedding in the respiratory tract of the subject.

### Reduction of inflammation

Chronic inflammation causes structural changes and narrowing of the small airways, which contributes to the signs of COPD, and airway injury and remodeling that lead to irreversible dilation of the bronchi in NCFB. Increased inflammation and resulting damage may increase the risk of an acute exacerbation. The composition of the invention may reduce inflammation in the subject, and so it is particularly suitable for use in the prevention or treatment of an acute exacerbation, typically in a subject with COPD or NCFB.

In one embodiment, the composition of the invention reduces inflammation in the subject, typically local inflammation, e.g. respiratory tract inflammation. In particular, the composition reduces pathogen-induced inflammation, particularly pathogen-induced inflammation in the respiratory tract of the subject.

The inflammation may be characterized by an increased level of one or more pro-inflammatory cytokines, such as IL-1β and/or IL-6 and/or IL-8. Therefore, in a specific embodiment the composition of the invention reduces the level of IL-1β and/or IL-6 and/or IL-8, in particular in the mucus layer of the respiratory tract.

The level of one or more cytokines (e.g. the level of IL-1β and/or IL-6 and/or IL-8) in the mucus layer of the respiratory tract can be quantified by analysis of the level in sputum produced by the subject. Cytokine concentrations in sputum can be quantified according to standard methods, for example by rate nephelometry [14] or ELISA (e.g. in the modes of the invention described below).

### Polyclonal immunoglobulin

The invention pertains to the use of a composition comprising human plasma-derived Immunoglobulin G for the prevention or treatment of an acute exacerbation in a subject with a chronic lung disease as defined in claim 1. Such polyclonal immunoglobulins have been used successfully for the treatment of infectious diseases, as replacement therapy in subjects with primary immunodeficiency disorders, and for the prophylaxis and treatment of various inflammatory and autoimmune conditions, as well as certain neurological disorders.

These polyclonal immunoglobulin preparations were developed for systemic administration, and largely comprise IgG. Currently, these preparations are derived from pooled plasma of thousands of healthy donors (1,000 to 60,000 donors) and contain both specific and natural antibodies, reflecting the cumulative antigen experience of the donor population. This large spectrum of specific and natural antibodies can recognize a broad range of antigens (e.g. pathogens, foreign antigens and self/autoantigens).

Generally polyclonal immunoglobulins are administered intravenously or subcutaneously. Several commercial formulations are available for these administration routes.

The composition of the invention comprises polyclonal IgG. The polyclonal immunoglobulin is obtained from plasma of human donors. Preferably, the plasma from multiple donors is pooled in order to maximize the diversity of target antigen specificities, for example from more than 100 donors, preferably from more than 500 donors, even more preferably from more than 1,000 donors.

Typically, the plasma pools are subjected to ethanol fractionation, followed by several purification steps, such as further precipitation steps and/or column chromatography steps, as well as steps to inactivate and remove viral and other pathogens such as nanofiltration or solvent/detergent treatment, for example a method as shown in reference 16.

The polyclonal immunoglobulin is polyclonal IgG. The composition comprises polyclonal IgG. The composition may also comprise J-chain containing IgA and/or IgM, combined with secretory component, as disclosed in WO2013/132052.

### IgG

The invention relates to compositions comprising polyclonal immunoglobulin as defined in claim 1 for use in the prevention or treatment of an acute exacerbation in a subject with the chronic lung disease COPD or NCFB. Surprisingly, it was found by the present inventors that large immune complexes are formed between IgG and *Pseudomonas aeruginosa.* Antigen binding by immunoglobulin is largely dependent on the target antigen-binding (Fab) domain. As IgG is only divalent with respect to the Fab domain, such aggregates (immune complexes) between *Pseudomonas aeruginosa* and IgG were unexpected. Therefore, the composition of the invention comprises polyclonal human plasma-derived IgG. In one embodiment, the polyclonal immunoglobulin is at least 95% IgG, preferably at least 98% IgG. The polyclonal IgG is particularly suitable for use in the prevention or treatment of an acute exacerbation in a subject with the chronic lung disease COPD or NCFB, by treating and/or preventing one or more respiratory tract infections.

One explanation for the unexpected formation of immune complexes of IgG and *Pseudomonas aeruginosa* is that IgG may additionally bind *Pseudomonas aeruginosa* outside of the Fab regions, potentially through its sugars. IgG may therefore be surprisingly more potent at signaling *Pseudomonas aeruginosa* to the immune system than expected. Consequently, in a specific embodiment, a composition of the invention comprising IgG is used for the prevention or treatment of an acute exacerbation in a subject with a chronic lung disease, typically COPD and/or NCFB, wherein the subject has a concurrent *Pseudomonas aeruginosa* infection. In another embodiment, a composition of the invention comprising IgG is administered to a subject to prevent respiratory tract infection with *Pseudomonas aeruginosa.* Thus, in a preferred embodiment, a composition of the invention comprising IgG is used for the prevention of an acute exacerbation, wherein the composition is administered as maintenance therapy. This composition is particularly useful for maintenance therapy in a subject with the chronic lung disease COPD or NCFB, because *Pseudomonas aeruginosa* is an opportunistic pathogen that can affect subjects with impaired lung defenses, such as patients with COPD or NCFB. In particular, it has been described to be one of the most harmful bacteria found in subjects with COPD and during acute exacerbations of COPD [7].

Normal human IgG can be obtained with a purity of at least 95% IgG, which means that 95% of the polyclonal Ig is IgG. Thus, in one embodiment, the IgG contained in the composition of the invention generally has a purity of at least 95% IgG, preferably at least 96% IgG, more preferably at least 98% IgG, for example at least 99% IgG.

Administration of a composition comprising IgA to a subject with a selective IgA deficiency may lead to anaphylaxis in the subject. Anaphylaxis is a serious allergic reaction that often starts rapidly and may lead to death of the subject. Accordingly, in some embodiments, the composition of the invention comprises only a minor amount of IgA, for example less than 200 µg/mL of IgA, preferably less than 25 µg/mL of IgA. These compositions are particularly suitable for administration to a subject, wherein the subject has a selective IgA deficiency. Moreover, selective IgA deficiencies do not have severe symptoms, and so a subject of the invention may not be aware that it has a selective IgA deficiency. Accordingly, these compositions are particularly useful for administration to a subject that is not aware of whether or not it has a selective IgA deficiency.

Thus, in a preferred embodiment, the composition of the invention comprises polyclonal immunoglobulin that is at least 98% IgG, and comprises less than 25 µg/mL of IgA.

In a specific embodiment, the composition for use in the invention is Privigen^{™}. Commercially available immunoglobulin formulations that can also be used according to the invention include: Bivigam^{™}, Clairyg^{™}, Flebogam^{™} 5%, Flebogamma^{™} DIF 5%, Gammagard^{™} Liquid 10%, Gammaplex^{™}, Gamunex^{™} 10%, IG Vena^{™} N, Intratect^{™}, Kiovig^{™}, Nanogam^{™}, Octagam^{™}, Octagam^{™} 10%, Polyglobin^{™} N10%, Sandoglobulin^{™} NF liquid, Vigam^{™} and IQYMUNE^{™}.

### Polyclonal immunoglobulin enriched for a specific antibody

The invention relates to a composition as described in claim 1 for use in the prevention and/or treatment of an acute exacerbation in a subject with the chronic lung disease COPD or NCFB. As described above, an acute exacerbation may be caused by a respiratory tract infection in the subject. In one embodiment, the composition of the invention is enriched for one or more antibodies specific for one or more particular pathogens (e.g. bacteria and/or virus) or potentially pathogenic microbes (e.g. bacteria and/or virus). Such a composition may be particularly useful because it has the effect of increasing the effective dosage of the immunoglobulin that is active against the microbe or pathogen, which will therefore have a greater therapeutic effect, or can achieve an equivalent therapeutic effect when a lower total dose of the composition is administered. In one embodiment, the composition of the invention is enriched for an antibody specific for a pathogen by supplementing the composition with monoclonal antibodies specific for the pathogen.

In one embodiment, the composition of the invention is enriched with antibodies specific for one or more of rhinovirus, influenza A, human metapneumovirus, RSV, coronavirus, influenza B, adenovirus, *Pseudomonas aeruginosa, Haemophilus influenza, Streptococcus pneumonia, Moraxella catarrhalis, Haemophilus parainfluenzae* and/or *Staphylococcus aureus.* Such a composition may be particularly useful because these pathogens are the most common cause of an acute exacerbation in a subject with a chronic obstructive respiratory disease, typically COPD and/or NCFB. Preferably, the composition of the invention is enriched for an antibody specific for *Pseudomonas aeruginosa,* which has been described to be one of the most harmful bacteria found in subjects with COPD and during exacerbations of COPD [7]. Preferably, the composition of the invention is enriched for an antibody specific for human rhinovirus, which is the most common viral infection to cause an acute exacerbation of COPD.

In one embodiment, the composition of the invention that is enriched for antibodies with specificity for particular pathogens can be obtained by supplementing a composition comprising polyclonal immunoglobulin with monoclonal Abs or a mixture of two or more monoclonal antibodies, with specificity for one or more pathogens selected from: rhinovirus, influenza A, human metapneumovirus, RSV, coronavirus, influenza B, adenovirus, *Pseudomonas aeruginosa, Haemophilus influenza, Streptococcus pneumonia, Moraxella catarrhalis, Haemophilus parainfluenzae* and/or *Staphylococcus aureus.*

In one embodiment, the composition of the invention that is enriched for antibodies with specificity for particular pathogens can be obtained by supplementing a composition comprising polyclonal immunoglobulin with polyclonal immunoglobulins obtained from a transgenic animal engineered to express human immunoglobulins following immunization with the particular pathogen.

In one embodiment, the composition of the invention that is enriched for antibodies with specificity for particular pathogens can be obtained by supplementing a composition comprising polyclonal immunoglobulin with several specific immunoglobulins obtained from screening a library of human antigen binding sites with the particular pathogen or antigens derived from the particular pathogen, and recombinantly producing pathogen-specific immunoglobulins with these antigen binding sites.

### Aerosols

The invention relates to a composition comprising polyclonal immunoglobulin as defined in claim 1 for use in the treatment or prevention of acute exacerbations in a patient with the chronic lung disease COPD or NCFB, wherein the composition is administered to the respiratory tract of the subject. Typically, the composition of the invention is administered to the respiratory tract of the subject as an aerosol. The aerosol may be generated by nebulizing a liquid aqueous composition comprising polyclonal immunoglobulin. Alternatively, the aerosol can be a dry powder aerosol, for example as produced by a dry powder inhalation system [17]. Alternatively, a soft mist inhaler, an aqueous droplet inhaler, or a pressurized metered dose inhaler, or any other device suitable for delivering immunoglobulin to the respiratory tract of a patient can be used.

### Liquid aqueous compositions

Liquid aqueous compositions are particularly suitable for nebulization to form an aerosol for administration to the respiratory tract of the subject. Therefore, the composition of the invention is generally in liquid aqueous form. Liquid aqueous compositions are liquid systems wherein the liquid carrier or solvent consists predominantly or completely of water. In specific cases, the liquid carrier can contain small fractions of one or more liquids which are at least partly miscible with water.

The invention relates to administration of the composition of the invention to the respiratory tract of the subject. For such administration to the respiratory tract, it is preferred to use high concentrations polyclonal immunoglobulin. Generally, high doses of polyclonal immunoglobulin are useful to increase efficacy, but it is also useful to minimize the volume to be administered as much as possible, for example when administered by nebulizer to keep the nebulization time as short as possible. Keeping nebulization time as short as possible is particularly useful for maintaining subject compliance. Thus, in one embodiment, the composition of the invention has a high concentration of polyclonal immunoglobulin, for example between about 20 and about 200 mg/mL. The concentration of the polyclonal immunoglobulin may range between 20 and 190 mg/mL, 20 and 180 mg/mL, 20 and 170 mg/mL, 20 and 160 mg/mL, 20 and 150 mg/mL, 30 and 200 mg/mL, 30 and 190 mg/mL, 30 and 180 mg/mL, 30 and 170 mg/mL, 30 and 160 mg/mL, 30 and 150 mg/mL, 40 and 200 mg/mL, 40 and 190 mg/mL, 40 and 180 mg/mL, 40 and 170 mg/mL, 40 and 160 mg/mL, 40 and 150 mg/mL. Polyclonal immunoglobulin concentrations that are suitable for the composition of the invention range between 20 and 140 mg/mL, 20 and 130 mg/mL, 20 and 120 mg/mL, 30 and 140 mg/mL, 30 and 130 mg/mL, 30 and 120 mg/mL, 40 and 140 mg/mL, 40 and 130 mg/mL, 40 and 120 mg/mL, 50 and 140 mg/mL, 50 and 130 mg/mL or 50 and 120 mg/mL; in particular, the concentration of the polyclonal immunoglobulin is about 50 mg/mL, about 60 mg/mL, about 70 mg/mL, about 80 mg/mL, about 90 mg/mL, about 100 mg/mL, about 110 mg/mL, or about 120 mg/mL.

Relatively high concentrations are important to enable low fill volumes and short nebulization times and, thus, ensure therapeutic efficiency of the treatment. In a specific preferred embodiment, the composition comprises polyclonal IgG at a concentration of about 50 mg/mL to about 100 mg/mL. Most preferably, the composition comprises polyclonal IgG at a concentration of about 100 mg/mL.

Typically, a liquid aqueous composition of the invention contains one or more stabilizers. A commonly encountered issue when formulating liquid immunoglobulin formulations is that the immunoglobulins tend to aggregate and form precipitates if not sufficiently stabilized with appropriate additives. Accordingly, in one embodiment, the composition of the invention comprises a stabilizer, for example an amino acid, such as proline, glycine and histidine, or a saccharide, or a sugar alcohol, or a protein, such as albumin, or a combination thereof. Each of these additives are known to stabilize immunoglobulins in liquid aqueous formulations and may be used in the liquid aqueous composition of the invention. In a specific embodiment, the composition of the invention comprises a stabilizer, wherein the stabilizer is proline, glycine or histidine, preferably proline.

An increase of the immunoglobulin concentration in a liquid aqueous composition results in a non-linear increase of viscosity. To avoid nebulization issues caused by high viscosity, it has been found that proline is particularly suitable as a stabilizer, since a relatively low viscosity of the composition of the invention can be achieved even if the concentration of polyclonal immunoglobulin is high, as disclosed in WO2011/095543. Proline provides on the one hand the desired stability of polyclonal immunoglobulin in a liquid aqueous composition, and on the other hand it reduces the viscosity of the composition, thus allowing the nebulization of a small liquid volume with a high polyclonal immunoglobulin concentration, which results in a fast and efficacious treatment by nebulization. Accordingly, in a specific embodiment, the composition of the invention comprises proline, in particular when the composition of the invention is in liquid aqueous form.

L-proline is particularly suitable for use in the composition of the invention because it is normally present in the human body and has a very favorable toxicity profile. The safety of L-proline has been investigated in repeated-dose toxicity studies, reproduction toxicity studies, mutagenicity studies and safety pharmacology studies, and no adverse effects were noted. Therefore, in a preferred embodiment, the composition of the invention comprises L-proline. Generally, the composition of the invention comprises proline, preferably L-proline, in the range of from about 10 to about 1000 mmol/L, for example from about 100 to about 500 mmol/L, in particular about 250 mmol/L.

In a preferred embodiment, the composition of the invention contains about 210-290 mmol/L of L-proline, in particular 250 mmol/L of L-proline. In a specific embodiment, the composition comprises polyclonal IgG and about 250 mmol/L of L-proline.

In one embodiment, the viscosity of the liquid aqueous composition of the invention comprising polyclonal immunoglobulin and proline ranges between 1 mPa-s and 17 mPa-s (at a temperature of 20.0°C +/- 0.1°C). In a specific embodiment, the viscosity of a composition comprising 100 mg/mL polyclonal IgG and 250 mmol/L of L-proline is about 3 mPa-s at a temperature of 20.0°C +/- 0.1 °C.

Typically, the composition of the invention comprising polyclonal IgG and containing proline has a pH of 4.2 to 5.4, preferably 4.6 to 5.0, most preferably about 4.8, which further contributes to the high stability of the preparation.

The use of proline allows preparing a composition where stability of the formulation is increased and viscosity of the composition is reduced by using one single agent. This results in a composition which is particularly useful in methods for generating an aerosol with a mesh nebulizer.

A composition of the invention usually includes components in addition to the polyclonal immunoglobulin, e.g. it typically includes one or more further pharmaceutical carrier(s) and/or excipient(s). A discussion of such components is available in reference 18.

In one embodiment, the composition of the invention also comprises pharmaceutically acceptable excipients, which serve to optimize the characteristics of the composition and/or the characteristics of the aerosol. Examples of such excipients are excipients for adjusting or buffering the pH, excipients for adjusting osmolality, antioxidants, surfactants, excipients for sustained release or prolonged local retention, taste-masking agents, sweeteners, and flavors. These excipients are used to obtain an optimal pH, osmolality, viscosity, surface tension and taste, which support the formulation stability, the aerosolization, the tolerability and/or the efficacy of the formulation upon inhalation.

The liquid aqueous compositions of the invention typically have a surface tension of about 60 to 75 mLM/m, preferably about 64 to 71 mLM/m. Surfactants can be added to the composition of the invention. These can help to control the rate of aggregation of polyclonal immunoglobulin in the composition (i.e. during storage and in the reservoir) and during nebulization (i.e. during and after passing the mesh of the nebulizer), thereby having an influence on the activity of the polyclonal immunoglobulin, in the aerosol. Therefore, in one embodiment, the composition of the invention is a liquid aqueous composition comprising a surfactant, for example a polysorbate, such as polysorbate 80.

### Nebulization

The invention involves administration of the composition to the respiratory tract of the subject. The composition of the invention can be administered to the respiratory tract of the subject as an aerosol generated by nebulization of a liquid aqueous composition of the invention using a nebulizer.

A nebulizer is a device which is capable of aerosolizing a liquid material into a dispersed liquid phase. An aerosol is a system comprising a continuous gas phase and, dispersed therein, a discontinuous or dispersed phase of solid or liquid particles, typically liquid particles when generated by nebulization of a liquid aqueous composition.

The liquid aqueous composition of the invention can be nebulized by mesh nebulizer or ultrasonic nebulizer or jet nebulizer, or any other device capable of nebulizing the composition of the invention. In one embodiment, a mesh nebulizer may be used to generate the aerosol for administration to a subject. For example, mesh nebulizers and generated aerosols as disclosed in WO 2015/150510, may be used.

A dispersed liquid phase (aerosol) essentially consists of liquid droplets. The droplets of the dispersed phase comprise polyclonal Ig, e.g. IgG, IgA, IgM or combinations thereof, in a liquid environment. The liquid environment is mainly an aqueous phase, with or without further excipients as described further below. It will be understood by the person skilled in the art, that the features and preferences with respect to the liquid composition, as disclosed herein, may also be applied to the dispersed phase of the aerosol generated therefrom and vice versa.

Two values can be determined experimentally and may be useful to describe the particle size or droplet size of the generated aerosol: the mass median diameter (MMD) and the mass median aerodynamic diameter (MMAD). The difference between the two values is that the MMAD is normalized to the density of water (equivalent aerodynamic).

The MMAD may be measured by an impactor, for example the Anderson Cascade Impactor (ACI) or the Next Generation Impactor (NGI). Alternatively, laser diffraction methods may be used, for example the Malvern MasterSizer X^{™}, to measure the MMD.

The dispersed phase of the aerosol generated by the method of the present disclosure typically exhibits a particle size, e.g. the MMD of preferably less than 10 µm, preferably from about 1 to about 6 µm, more preferably from about 1.5 to about 5 µm and even more preferably from about 2 to about 4.5 µm. Alternatively, the particle size may have a MMAD of preferably less than 10 µm, preferably from about 1 to about 6 µm, more preferably from about 1.5 to about 5 µm and even more preferably from about 2 to about 4.5 µm. Another parameter describing the dispersed phase of the aerosol is the particle size distribution of the aerosolized liquid particles or droplets. The geometric standard deviation (GSD) is an often used measure for the broadness of the particle or droplet size distribution of generated aerosol particles or droplets. The selection of the precise MMD within the above described range should take the target region or tissue for deposition of the aerosol into account. For example, the optimal droplet diameter will differ depending on whether oral, nasal or tracheal inhalation is intended, and whether upper and/or lower respiratory tract delivery (e.g. to the oropharynx, throat, trachea, bronchi, alveoli, lungs, nose, and/or paranasal sinuses) is focused upon. Additionally, the age dependent anatomic geometry (e.g. the nose, mouth or respiratory airway geometry) as well as the respiratory disease and condition of the subjects and their breathing pattern belong to the important factors determining the optimal particle size (e.g. MMD and GSD) for drug delivery to the lower or upper respiratory tract.

Generally, small airways, which are defined by an internal diameter lower than 2 mm, represent almost 99% of the lung volume and therefore play an important role in lung function. Alveoli are sites in the deep lungs where oxygen and carbon dioxide are exchanged with the blood. Inflammation in the alveoli induced by some viruses or bacteria leads to fluid secretion on site and directly affects oxygen uptake by the lungs. Therapeutic targeting of deep pulmonary airways with aerosols requires aerosols having an MMD below 5.0 µm, preferably below 4.0 µm, more preferably below 3.5 µm and even more preferably below 3.0 µm. Such MMD values are therefore envisaged for use in the invention.

For aerosol delivery to the respiratory tract, the aerosol has an MMD below 10.0 µm, preferably below 5.0 µm, more preferably below 3.3 µm, and even more preferably below 2.0 µm. Preferably, the MMD is (droplet sizes are) in the range from about 1.0 to about 5.0 µm and the size distribution has a GSD less than 2.2, preferably less than 2.0, more preferably less than 1.8 or even more preferably less than 1.6. Such particle size and particle size distribution parameters are particularly useful to achieve a high local drug concentration in the respiratory tract (e.g. lungs) of humans, including the bronchi and bronchioli, relative to the amount of drug which is aerosolized. In this context it must be considered that deep lung deposition requires smaller MMD's than deposition in the central airways of adults and children and for infants and babies even smaller droplet sizes (MMD's) in the range from about 1.0 to about 3.3 µm are more preferred and the range below 2.0 µm is even more preferred. Thus, in aerosol therapy it is common to evaluate the fraction of droplets smaller than 5 µm (representing the fraction that is respirable by an adult) and smaller than 3.3 µm (representing the fraction that is respirable by a child or is deposited in the deeper lungs of an adult). Also, the fraction of droplets smaller than 2 µm is often evaluated as it represents the fraction of the aerosol that could optimally reach terminal bronchioles and alveoli of adults and children and can penetrate the lungs of infants and babies.

In the invention, the fraction of droplets having a particle size smaller than 5 µm is preferably greater than 65%, more preferably greater than 70% and even more preferably greater than 80%. The fraction of droplets having a particle size smaller than 3.3 µm is preferably greater than 25%, more preferably greater than 30%, even more preferably greater than 35% and still more preferably greater than 40%. The fraction of droplets having a particle size smaller than 2 µm is preferably greater than 4%, more preferably greater than 6% and even more preferably greater than 8%.

The aerosol can also be characterized by its delivered dose (DD) as determined in breath simulation experiments. The delivered dose can be used to calculate the respirable dose (RD), e.g. on the basis of the respirable fraction (RF) measured by laser diffraction (e.g. Malvern MasterSizer X^{™}) or using an impactor (e.g. Anderson Cascade Impactor - ACI, or Next Generation Impactor - NGI). When applying the method of the present disclosure in a breath simulation experiment (e.g. using a breathing simulator like BRS3000 from Copley or Compass II^{™} from PARI) with an adult breathing pattern (sinusoidal flow, 500 mL tidal volume, 15 breaths/min), and filling 2 mL of composition (e.g. 200 mg Ig, 200 mg IgG, 200mg IgA, 200mg IgM or combinations thereof) into the mesh nebulizer, the delivered dose (DD) is preferably higher than 40% (80 mg Ig, e.g. IgG, IgA, IgM or combinations thereof), more preferably higher than 45% (90 mg Ig, e.g. IgG, IgA, IgM or combinations thereof) and even more preferably higher than 50% (100 mg Ig, e.g. IgG, IgA, IgM or combinations thereof).

For the treatment of the upper airways, in particular the nose, nasal and/or sinonasal mucosa, osteomeatal complex, and paranasal cavities, an MMD below about 5.0 µm, or below about 4.5 µm, or below about 4.0 µm, or below about 3.3 or below about 3.0 µm is particularly suitable.

The suitability of the generated aerosol for application to the upper airways can be evaluated in nasal inhalation models such as the human nasal cast model described in WO2009/027095. For aerosol delivery to the nose, e.g. the Sinus^{™} device (jet nebulizer) from PARI and also a mesh nebulizer (prototypes of Vibrent^{™} technology) exist.

The nebulizer used in the invention may be a mesh nebulizer. Preferably, the mesh nebulizer is a vibrating membrane nebulizer. Nebulizers of the latter type comprise a reservoir in which the liquid for the nebulization is filled. When operating the nebulizer, the liquid is fed to a mesh that is made to oscillate, i.e. vibrate (e.g. by means of a piezoelectric element). The liquid present at one side of the vibrating mesh is hereby transported through openings in the vibrating mesh (also referred to as "pores" or "holes") and takes the form of an aerosol on the other side of the vibrating mesh, (e.g. eFlow rapid and eRapid from PARI, HL100 from Health and Life as well as AeronebGo and AeronebSolo from Aerogen). Such nebulizers may be referred to as "active membrane nebulizers".

In other useful mesh nebulizers, the composition can be nebulized by vibrating the liquid rather than the membrane. Such an oscillating fluid mesh nebulizer comprises a reservoir in which the liquid to be nebulized is filled. When operating the nebulizer, the liquid is fed to a membrane via a liquid feed system that is made to oscillate (i.e. vibrate, e.g. by means of a piezoelectric element). This liquid feed system could be the vibrating back wall of the reservoir (e.g. AerovectRx^{™} Technology, Pfeifer Technology) or a vibrating liquid transporting slider (e.g. I-Neb^{™} device from Respironics, or U22^{™} device from Omron). These nebulizers may be referred to as "passive mesh nebulizers".

Different membrane types are available for the nebulization of liquids with a mesh nebulizer. These membranes are characterized by different pore sizes which generate aerosols with different droplet sizes (MMD's and GSD's). Depending on the characteristics of the composition and the desired aerosol characteristics, different membrane types (i.e. different modified mesh nebulizers or aerosol generators) can be used. In the invention, it is preferred to use membrane types which generate an aerosol with an MMD in the range of 2.0 µm to 5.0 µm, preferably in the range of 3.0 µm to 4.9 µm and more preferably in the range of 3.4 µm to 4.5 µm. In another embodiment of the invention, it is preferred to use membrane types built in aerosol generator devices which generate an aerosol, e.g. isotonic saline (NaCl 0.9%), with an MMD in the range of 2.8 µm to 5.5 µm, preferably in the range of 3.3 µm to 5.0 µm, and more preferably in the range 3.3 µm to 4.4 µm. In another embodiment of the invention, it is preferred to use membrane types built in aerosol generator devices which generate an aerosol, e.g. isotonic saline, with an MMD in the range of 2.8 µm to 5.5 µm, preferably in the range of 2.9 µm to 5.0 µm and more preferably in the range of 3.8 µm to 5.0 µm.

If the treatment is intended for targeting the lower respiratory tract such as the bronchi or the deep lungs, it is particularly preferred that a piezoelectric perforated mesh-type nebulizer is selected for generating the aerosol. Examples of suitable nebulizers include the passive mesh nebulizer, such as I-Neb^{™}, U22^{™} U1^{™}, Micro Air^{™}, the ultrasonic nebulizer, for example Multisonic^{™}, and/or active mesh nebulizer, such as HL100^{™}, Respimate^{™}, eFlow^{™} Technology nebulizers, AeroNeb^{™}, AeroNeb Pro^{™}, AeronebGo^{™}, and AeroDose^{™} device families as well as the prototype Pfeifer, Chrysalis (Philip Morris) or AerovectRx^{™} devices. A particularly preferred nebulizer for targeting the drug to the lower respiratory tract is a vibrating perforated membrane nebulizer or so called active mesh nebulizer, such as for example the eFlow^{™} nebulizer (electronic vibrating membrane nebulizer available from PARI, Germany). Alternatively, a passive mesh nebulizer may be used, for example U22^{™} or U1^{™} from Omron or a nebulizer based on the Telemaq.fr technique or the Ing. Erich Pfeiffer GmbH technique.

A preferred mesh nebulizer for targeting the upper respiratory tract is a nebulizer which generates the aerosol via a perforated vibrating membrane principle, such as a modified investigational membrane nebulizer using the eFlow^{™} technology, but which is also capable of emitting a pulsating air flow so that the generated aerosol cloud pulsates (i.e. undergoes fluctuations in pressure) at the desired location or during transporting the aerosol cloud to the desired location (e.g. sinonasal or paranasal sinuses). This type of nebulizer has a nose piece for directing the flow transporting the aerosol cloud into the nose. Aerosols delivered by such a modified electronic nebulizer can reach sinonasal or paranasal cavities much better than when the aerosol is delivered in a continuous (non-pulsating) mode. The pulsating pressure waves achieve a more intensive ventilation of the sinuses so that a concomitantly applied aerosol is better distributed and deposited in these cavities.

More particularly, a preferred nebulizer for targeting the upper respiratory tract of a subject is a nebulizer adapted for generating an aerosol at an effective flow rate of less than about 5 liters/min and for simultaneously operating means for effecting a pressure pulsation of the aerosol at a frequency in the range from about 10 to about 90 Hz, wherein the effective flow rate is the flow rate of the aerosol as it enters the respiratory system of the subject. Examples of such electronic nebulization devices are disclosed in WO2009/027095.

In a preferred embodiment of the invention, the nebulizer for targeting the upper respiratory tract is a nebulizer which uses a transportation flow that can be interrupted when the aerosol cloud reaches the desired location and then starts the pulsation of the aerosol cloud, e.g. in an alternating mode. The details are described in WO2010/097119 and WO2011/134940.

Whether adapted for pulmonary or sinonasal delivery, the nebulizer should preferably be selected or adapted to be capable of aerosolizing a unit dose at a preferred output rate. A unit dose is defined herein as a volume of the liquid aqueous composition comprising the effective amount of active compound, i.e. Ig, IgG, IgA, IgM or combinations thereof, designated to be administered during a single administration. Preferably, the nebulizer can deliver such a unit dose at a rate of at least 0.1 mL/min or, assuming that the relative density of the composition will normally be around 1, at a rate of at least 100 mg/min. More preferably, the nebulizer is capable of generating an output rate of at least 0.4 mL/min or 400 mg/min, respectively. In further embodiments, the liquid output rates of the nebulizer or the aerosol generator are at least 0.50 mL/min, preferably at least 0.55 mL/min, more preferably at least 0.60 mL/min, even more preferably at least 0.65 mL/min, and most preferably at least 0.7 mL/min, such devices called aerosol generator with a high output or high output rate. Preferably, the liquid output rate ranges between about 0.35 and about 1.0 mL/min or between about 350 and about 1000 mg/min; preferably the liquid output rate ranges between about 0.5 and about 0.90 mL/min or between about 500 and about 800 mg/min. Liquid output rate means the amount of liquid composition nebulized per time unit. The liquid may comprise an active compound, drug, Ig, IgG, IgA, IgM or combinations thereof and/or a surrogate such as sodium chloride 0.9%.

The output rate of the nebulizer should typically be selected to achieve a short nebulization time of the liquid composition. Obviously, the nebulization time will depend on the volume of the composition which is to be aerosolized and on the output rate. Preferably, the nebulizer should be selected or adapted to be capable of aerosolizing a volume of the liquid composition comprising an effective dose of polyclonal Ig, e.g. IgG, IgA, IgM or combinations thereof, within not more than 20 minutes. More preferably, the nebulization time for a unit dose is not more than 15 minutes. In a further embodiment, the nebulizer is selected or adapted to enable a nebulization time per unit dose of not more than 10 minutes, and more preferably not more than 6 minutes and even more preferably not more than 3 minutes. Presently most preferred is a nebulization time in the range from 0.5 to 5 minutes.

The volume of the composition that is nebulized according to the invention is preferably low in order to allow short nebulization times. The volume, also referred to as the volume of a dose, or a dose unit volume, or a unit dose volume, should be understood as the volume which is intended for being used for one single administration or nebulizer therapy session. Specifically, the volume may be in the range from 0.3 mL to 6.0 mL, preferably 0.5 mL to 4.0 mL, or more preferably 1.0 mL to about 3.0 mL, or even more preferably about 2.0 mL. In case a residual volume is desired or helpful, this residual volume should be less than 1.0 mL, more preferably less than 0.5 mL, and most preferably less than 0.3 mL. The effectively nebulized volume is then preferably in the range from 0.2 to 3.0 mL or 0.5 to 2.5 mL, or more preferably in the range from 0.75 to 2.5 mL or 1.0 to 2.5 mL.

Preferably, the nebulizer is adapted to generate an aerosol where a major fraction of the loaded dose of liquid composition is delivered as aerosol, i.e. to have a high output. More specifically, the nebulizer is adapted to generate an aerosol which contains at least 50% of the dose of the Ig, e.g. IgG, IgA, IgM or combinations thereof, in the composition, or, in other words, which emits at least 50% of the liquid composition filled in the reservoir. Especially in comparison with monoclonal antibodies, of which the doses do not need to be as high due to their specificity, it is important to select a nebulizer which can generate such high output of polyclonal Ig, e.g. IgG, IgA, IgM or combinations thereof. It was found that a mesh nebulizer as used in the method of the invention is capable of generating an aerosol of a polyclonal Ig, e.g. IgG, IgA, IgM or combinations thereof, composition with a particularly high output.

### Dry powder inhalation

The composition of the invention may also be a dry powder. Various forms of dry powder inhalers are available, such as capsule and multi-dose dry powder inhalers, single dosage forms such as a rotary inhaler, multi-dose such as Accuhaler and disc inhalers. Dry powder inhalers may be advantageous by providing an easy to use, fast inhalation system, suitable for more frequent use.

### Dosing

The composition of the invention is for use in the prevention or treatment of an acute exacerbation.

In one embodiment, the composition of the invention is for use in the prevention of an acute exacerbation in a subject with a chronic lung disease (typically COPD or NCFB), wherein the composition is administered as maintenance therapy. Maintenance therapy means that once therapy starts, the subject continues with the therapy for an extended period of time. For example, the therapy continues for at least six months. Typically, the therapy continues for at least one year.

The composition of the invention is administered to the respiratory tract of the subject, typically as an aerosol. In particular, the aerosol may be generated from a liquid aqueous composition using a nebulizer. Suitable liquid aqueous compositions are set out above. In a preferred embodiment, the liquid aqueous composition has a polyclonal immunoglobulin (e.g. IgG, IgA, IgM or combinations thereof) concentration from about 50 mg/mL to about 150 mg/mL, for example about 100 mg/mL.

For administration to the respiratory tract of the subject of the invention, the liquid aqueous composition used to generate the aerosol may be administered in a volume of 2-10 mL.

For use in the treatment or prevention of acute exacerbations (typically prevention), for example acute exacerbations of COPD or NCFB, the composition may be administered once every 48 hours, once every 24 hours or once every 12 hours during the therapy. In a specific embodiment, the composition of the invention is administered once every 12 hours. In a specific embodiment, the composition of the invention is administered every 24 hours. In a specific embodiment, the composition of the invention is administered every 48 hours.

For use in the prevention or treatment of acute exacerbations (typically prevention), for example acute exacerbations of COPD or NCFB, a dose of about 0.01 g to about 1.5 g of polyclonal immunoglobulin is used. A particularly suitable dose of the composition of the invention is from about 0.1 g to about 1.5 g of polyclonal immunoglobulin, for example a dose of from about 0.2 g to about 1 g, in particular, the dose is about 0.2 g. In a specific embodiment, a dose of about 0.2g is administered once per day.

Such doses may be adjusted, depending on factors that may increase the risk of acute exacerbations, for example the risk of respiratory tract infection. By way of example, the dose and/or frequency of administration according to the invention may be increased during the fall and winter months, in particular during the winter months.

In another embodiment, the aerosol may be generated from a dry composition using a dry powder inhaler. Typically, the dose delivered in one administration may be relatively low, such as around 0.5 mg, but the subject may use multiple inhalations per day, for example one to about twenty, preferably two to about fifteen, inhalations during a day.

### Seasonal administration

The therapy (prevention or treatment) of the invention is particularly useful during colder weather, which is associated with an increase in the rate of respiratory tract infections and acute exacerbations in subjects with chronic lung diseases, typically COPD and/or NCFB. In one embodiment, the composition is administered during the fall and/or winter months. In particular, the composition is administered during the winter months. The incidence of acute exacerbations of COPD shows seasonal variation, with higher rates during fall and winter months [19]. This increase in acute exacerbations may be caused by increased rates of respiratory tract infections, for example rhinovirus infections. Accordingly, administering the composition during the fall and winter months provides protection against such infections during a period of increased risk.

Such seasonal variation is thought to affect all subjects with COPD, and therefore this seasonal administration, for example in the fall and winter months, in particular the winter months, is useful for any subject with COPD characterized herein. The similarities between COPD and NCFB, in particular acute exacerbations that may be caused by respiratory tract infections, suggest that such seasonally varied administration would be expected to be useful for a subject with NCFB.

As used herein, the term "fall months" refers to those months commonly recognized as occurring during the fall or autumn. In the northern hemisphere, these months include September, October and November. In the southern hemisphere, these months include March, April and May.

As used herein, the term "winter months" refers to those months commonly recognized as occurring during winter. In the northern hemisphere, these months include October, November, December, January and February. In the southern hemisphere, these months include April, May, June, July and August.

### Combination therapy with antibiotics

The polyclonal immunoglobulin of the invention is particularly suitable for administration in combination with an antibiotic, for example to prevent or treat a bacterial respiratory tract infection in a subject with a chronic lung disease, typically COPD and/or NCFB.

In one embodiment, the composition of the invention is administered with an antibiotic during the acute phase of the bacterial infection while the subject is receiving antibiotic therapy, i.e. the composition is administered during the first two days, in particular during the first three days, during the first four days, or during the first five days of infection, in addition to standard antibiotic therapy.

### General

The term "comprising" encompasses "including" as well as "consisting" e.g. a composition "comprising" X may consist exclusively of X or may include something additional e.g. X + Y. The word "substantially" does not exclude "completely" e.g. a composition which is "substantially free" from Y may be completely free from Y. Where necessary, the word "substantially" may be omitted from the definition of the invention.

The term "about" in relation to a numerical value x is optional and means, for example, *x*±10%. The composition of the invention is a composition comprising polyclonal immunoglobulin. Unless specifically stated otherwise, an effect attributed to the composition is mediated by the polyclonal immunoglobulin, rather than an unspecified additional component.

Unless specifically stated, a process comprising a step of mixing two or more components does not require any specific order of mixing. Thus components can be mixed in any order. Where there are three components then two components can be combined with each other, and then the combination may be combined with the third component, *etc.*

### BRIEF DESCRIPTION OF THE DRAWINGS

The invention will now be illustrated in the following non-limiting examples, with reference to the following figures:

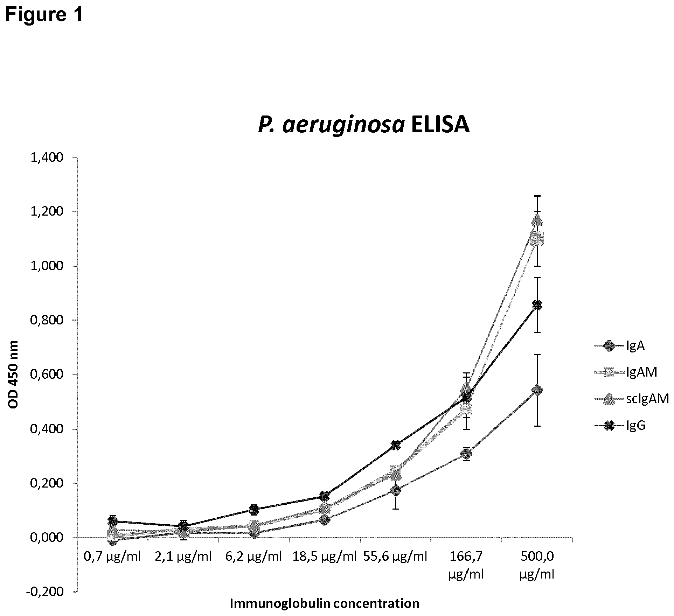

**Figure *1******.** Plasma-derived Ab formulations interact with Pseudomonas aeruginosa (PA).* Binding of increasing concentrations of plasma-derived Abs or secretory IgA/M to coated PA as determined by ELISA.

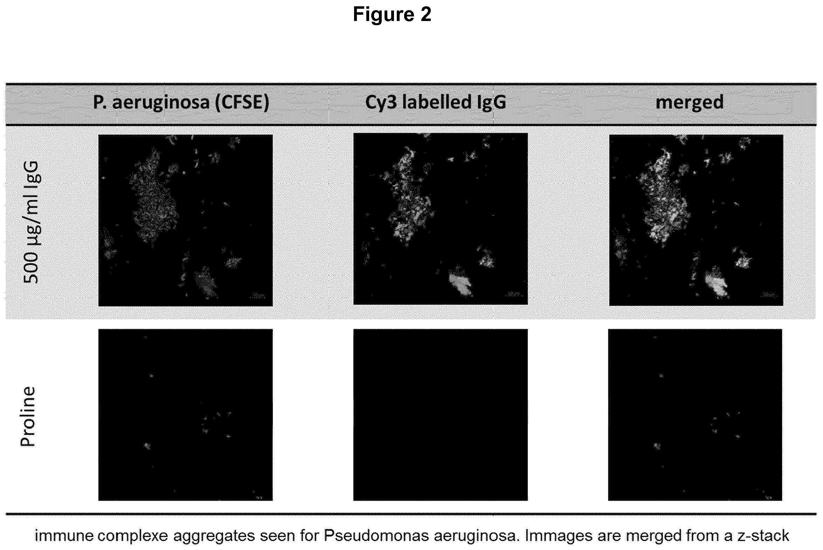

**Figure 2****.** *Association of plasma-derived IgG formulation with PA promotes agglutination.* Laser scanning confocal microscopy images of immune complexes of PA associated with plasma-derived IgG. Bacteria were labelled with CFSE and IgG with Cy3 dye. Images are representative of one observed field obtained from 5-10 observations from two independent slides.

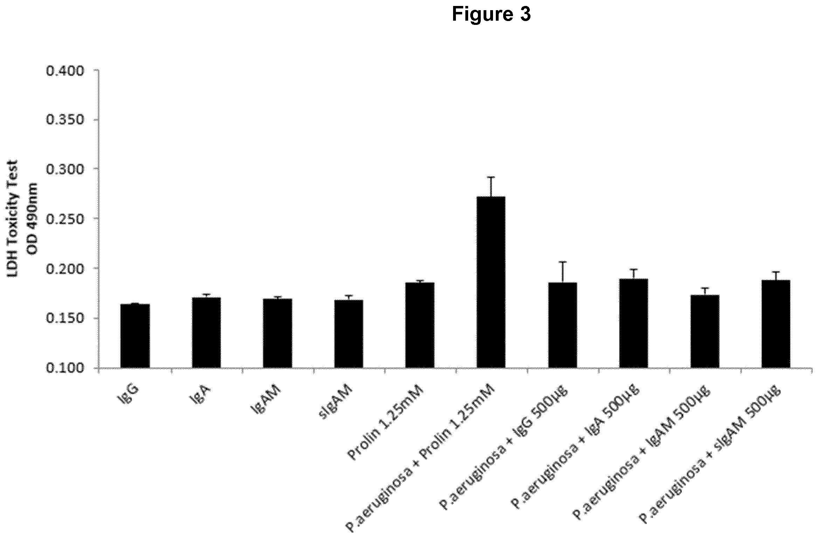

**Figure 3****.** *Plasma-derived immunoglobulin formulation PA-induced LDH tissue release.* Tissue damage was assessed by measuring LDH release in the basolateral medium of the MucilAir^{™}. Non-infected transwells receiving only the vehicle or the plasma-derived immunoglobulin formulations served as controls. PA-infected transwells served as positive controls. Data are representative of 3 independent experiments.

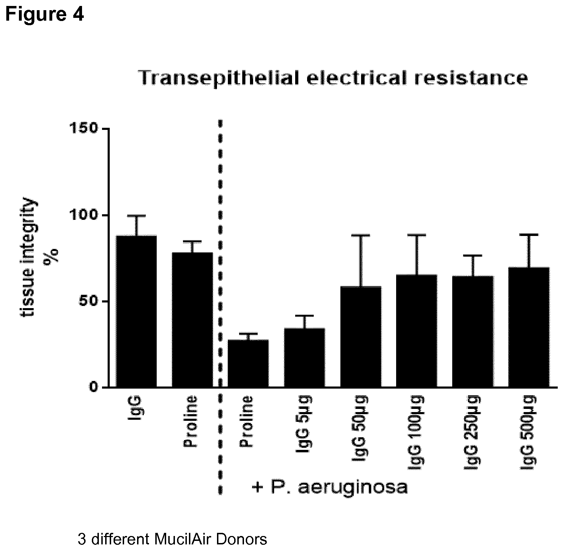

**Figure 4****.** *Plasma-derived IgG formulation prevents loss of trans-epithelial electrical resistance in a dose-dependent manner.* Tissue integrity was assessed by measuring trans-epithelial electrical resistance. Non-infected and proline treated transwells served as negative controls and PA-infected transwells as positive controls of tissue damage. Data are representative of 3 independent experiments.

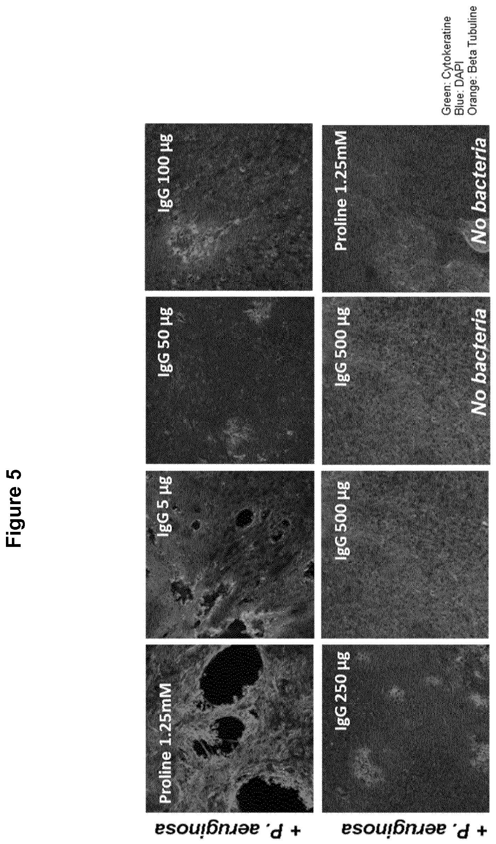

**Figure 5****.** *Plasma-derived IgG formulation prevent PA-induced tissue damages in* a *dose depend manner.* Laser scanning confocal microscopy images of paraffin-fixed MucilAir^{™} sections were acquired and analyzed for the expression of cytokeratine and beta-tubulin. Non-infected transwells receiving only the vehicle or IgG formulations served as controls. PA-infected transwells treated with vehicle served as positive controls. Data are representative of 3 independent experiments.

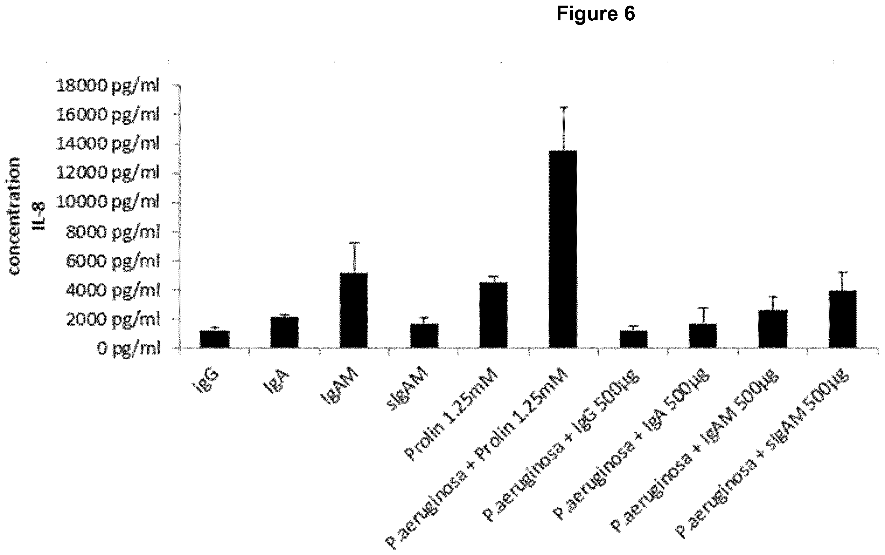

**Figure 6****.** *Plasma-derived immunoglobulin formulation reduces PA-induced IL-8 release by epithelial cells.* IL-8 was measured in the basolateral medium of the MucilAir^{™}. Non-infected transwells receiving only the vehicle or immunoglobulin formulations served as controls. PA-infected transwells treated with proline served as positive controls. Data are representative of 3 independent experiments.

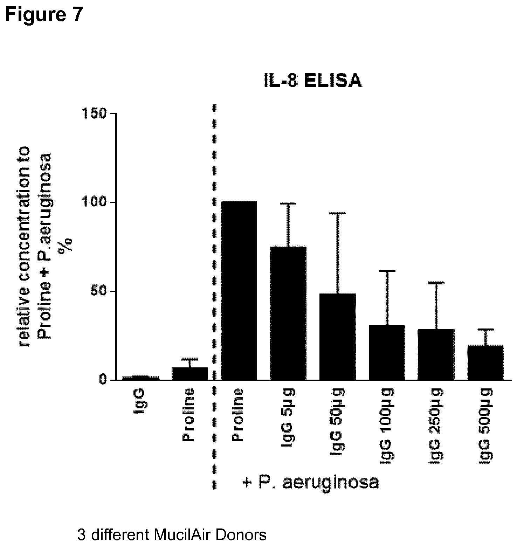

**Figure 7****.** *Plasma-derived IgG formulation reduces PA-induced IL-8 release by epithelial cells in a dose-dependent manner.* Relative concentrations of IL-8 secretion were calculated in regards to IL-8 secreted by MucilAir^{™} when exposed for 24h with 10 CFU of PA. Non-infected transwells receiving only the vehicle or immunoglobulin formulations served as controls. PA-infected transwells treated with proline served as positive controls. Data are representative of one experiment using MucilAir^{™} from 3 different donors per condition.

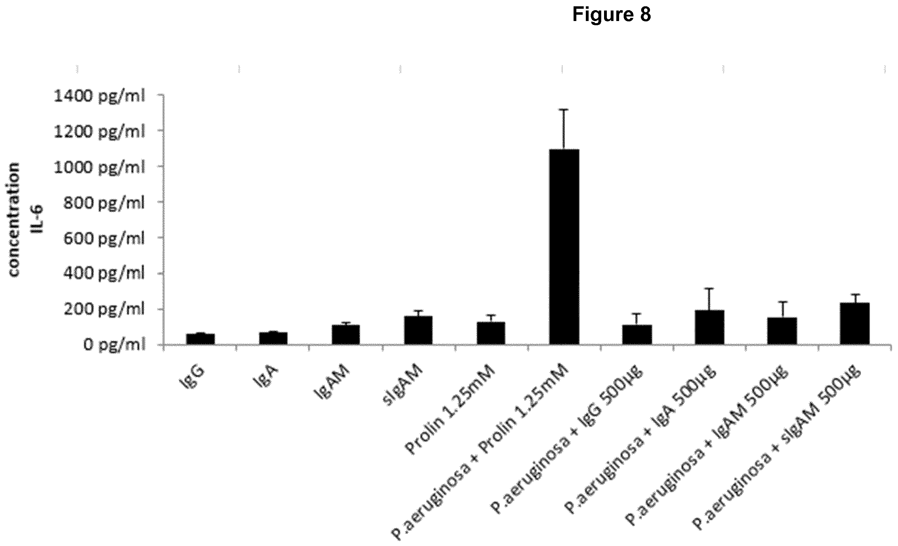

**Figure 8****.** *Plasma-derived Ab formulation reduces PA-induced IL-6 release by epithelial cells.* IL-6 was measured in the basolateral medium of the MucilAir^{™}. Non-infected transwells receiving only the vehicle or immunoglobulin formulations served as controls. PA-infected transwells treated with vehicle served as positive controls. Data are representative of 3 independent experiments.

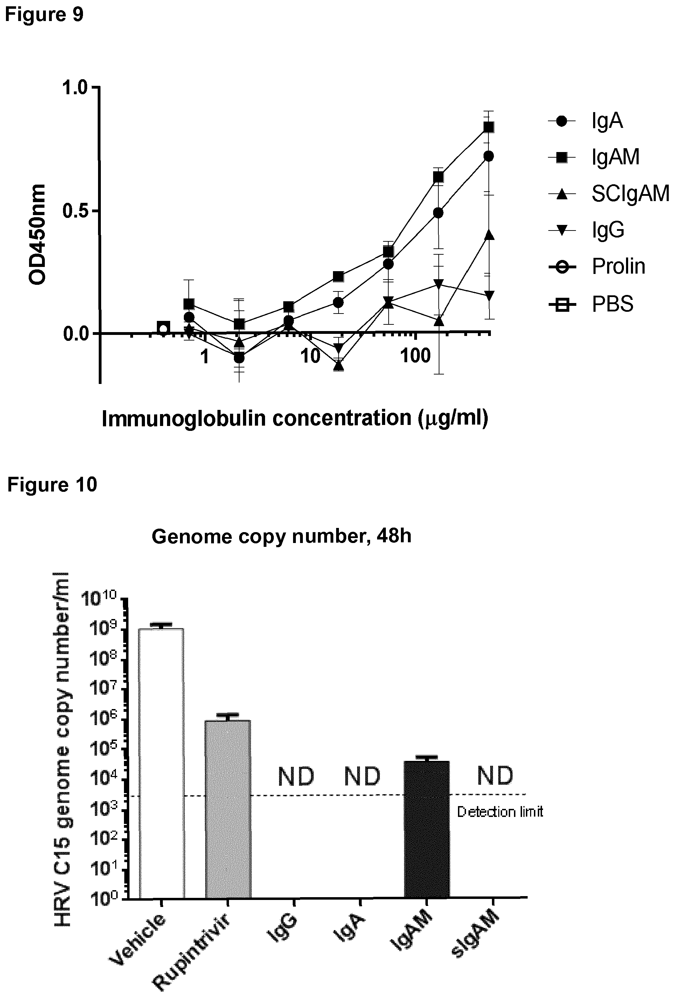

**Figure 9****.** *Plasma-derived Ab formulations interact with human rhinovirus* C15. Binding of increasing concentrations of plasma-derived Abs or secretory IgAM to coated HRV C15 as determined by ELISA.
**Figure 10****.** *Plasma-derived Abs reduce HRV shedding.* Copy number of HRV-C15 genome was measured in apical washes using q-PCR. HRV-infected transwells treated with proline served as positive controls of infection. For efficacy measurements, Rupintrivir treated transwells served as positive control.

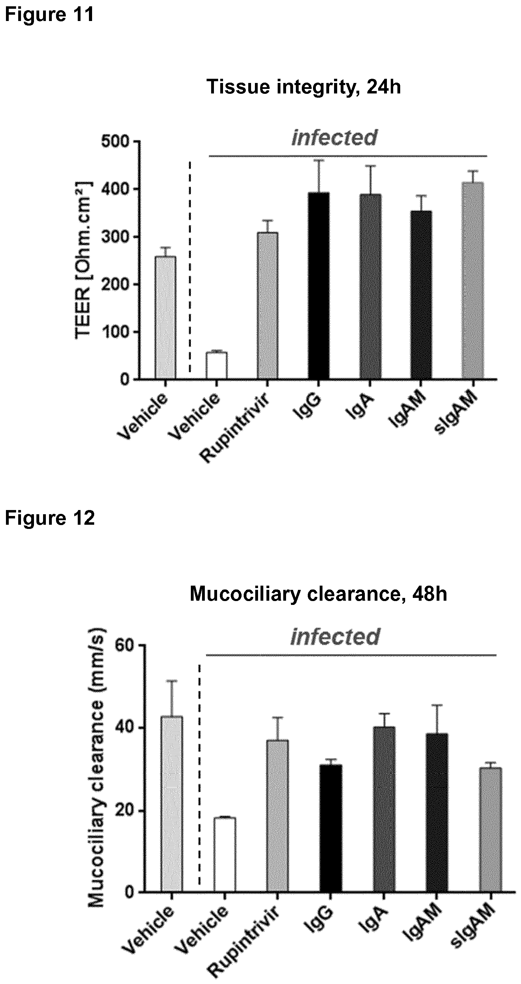

**Figure 11****.** *Plasma-derived Abs reduce HRV-induced tissue damage.* Tissue integrity was assessed by measuring trans-epithelial electrical resistance. Non-infected transwells treated with proline served as negative controls. HRV-infected transwells treated with proline served as positive controls of infection. For efficacy measurements, Rupintrivir treated transwells served as positive control.
**Figure 12****.** *Plasma-derived Abs prevent HRV-induced mucociliary clearance reduction.* Mucociliary clearance was assessed by measuring the speed of polystyrene microbeads of 30 µm diameter added on the apical surface of MucilAir^{™}. Non-infected transwells treated with proline served as negative controls. HRV-infected transwells treated with proline served as positive controls of infection. For efficacy measurements, Rupintrivir treated transwells served as positive control.

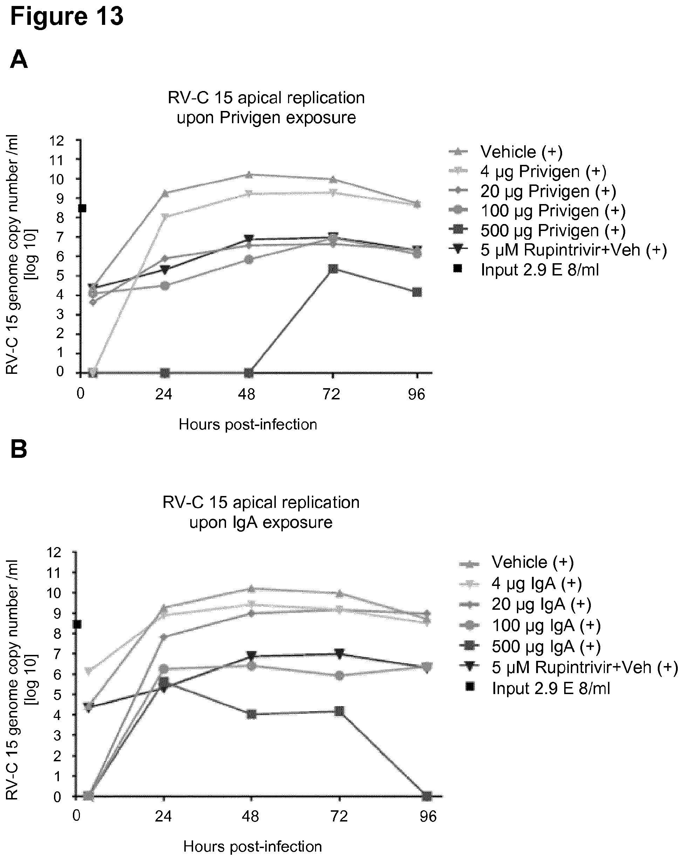

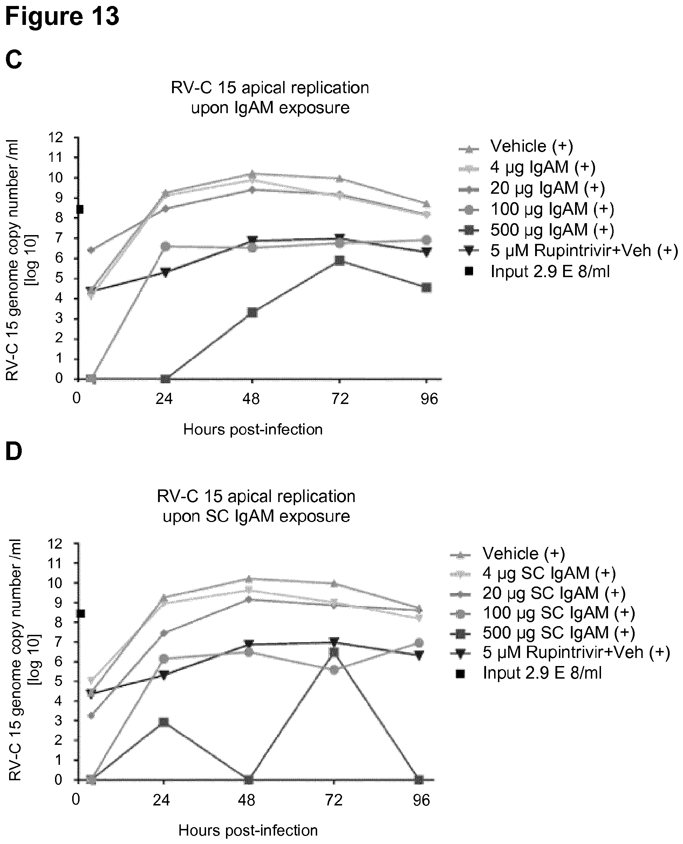

**Figure 13****.** Plasma-derived immunoglobulin formulations inhibit HRV proliferation in a dose-dependent manner. Copy number of HRV-C15 genome was measured in apical washes using q-PCR after treatment with 4 µg/well, 20 µg/well, 100 µg/well and 500 µg/well of the different immunoglobulin formulations. HRV-infected transwells treated with proline served as positive controls of infection. For efficacy measurements, Rupintrivir treated transwells served as positive control.

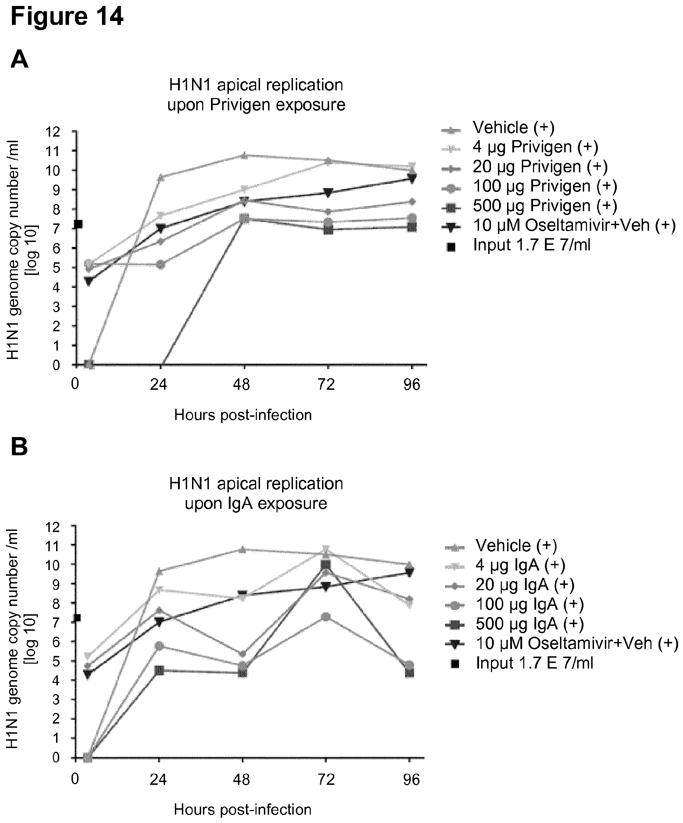

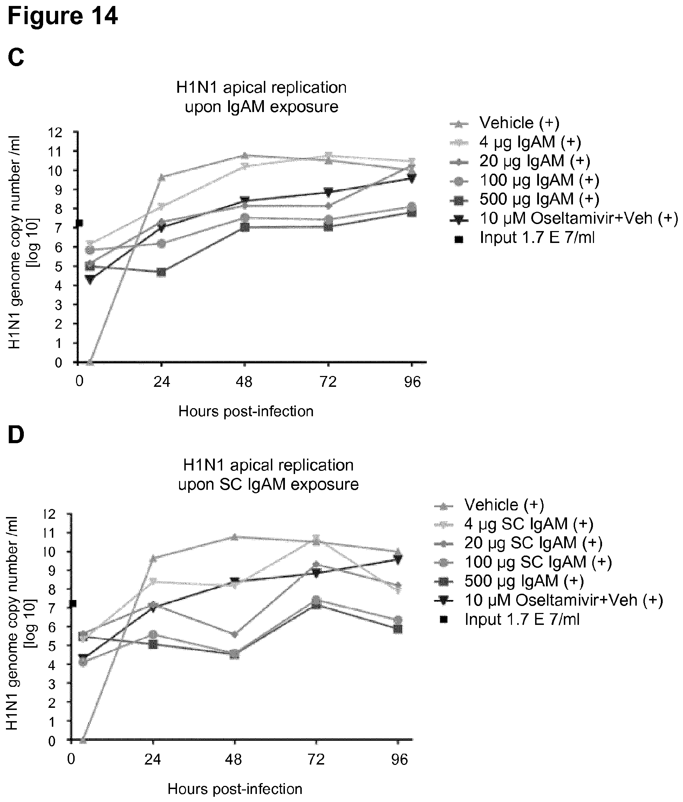

**Figure 14****.** Plasma-derived immunoglobulin formulations inhibit Influenza virus proliferation in a dose-dependent manner. Copy number of Influenza virus genome was measured in apical washes using q-PCR after treatment with 4 µg/well, 20 µg/well, 100 µg/well and 500 µg/well of the different immunoglobulin formulations. Influenza virus-infected transwells treated with proline served as positive controls of infection. For efficacy measurements, Oseltamivir-treated transwells served as positive control.

### MODES FOR CARRYING OUT THE INVENTION

The following non-limiting examples serve to illustrate the invention.

The studies encompassed in the examples below show that immunoglobulin delivered onto an airway tissue can have a combined antimicrobial (immune exclusion) and anti-inflammatory effect, and is therefore an attractive option for an effective treatment or prevention of an exacerbation, in particular an infection-related exacerbation, in subjects suffering from the chronic lung diseases COPD and NCFB. In particular, it is suitable for maintenance therapy in subjects with these diseases to prevent chronic infections and acute exacerbations.

Protection of mucosal surfaces against colonization and possible entry and invasion by microbes is provided by a combination of constitutive, non-specific substances (mucus, lysozyme and defensins), and also by specific immune mechanisms including secretory Igs (Slgs) at the humoral level [20;21]. In vivo, experimental and clinical resistance to infection can be correlated with specific secretory IgA (SlgA) antibodies (Abs) serving as an immunological barrier at mucosal surfaces [22;23]. It is thought that aggregation, immobilization and neutralization of pathogens at mucosal surfaces is facilitated by the multivalency of SIgA [24;25]. SIgM serving as a surrogate of SlgA in IgA-deficient individuals appears to act via a similar protective mechanism [26].

For a few pathogens such as Poliovirus, Salmonella, or influenza, protection against mucosal infection can be induced by active mucosal immunization with licensed vaccines. However, for the majority of mucosal pathogens no active mucosal vaccines are available. Alternatively, protective levels of Abs might directly be delivered to mucosal surfaces by passive immunization. In nature this occurs physiologically in many mammalian species by transfer of maternal antibodies to their offspring via milk [27]. Human and animal studies using passive mucosal immunization have demonstrated that plgA and SlgA antibody molecules administered by oral, intranasal, intrauterine or lung instillation can prevent, diminish, or cure bacterial and viral infections [28]. However, the secretory form of IgA naturally found at mucosal surfaces was rarely used, and large scale production of SlgA is not possible to date. Construction of SIgA with biotechnological methods is challenging but such molecules could have important clinical applications [29]. The same also applies to secretory component-containing IgM.

Plasma-derived immunoglobulins have been used for many decades to protect patients with immunodeficiencies from potentially lethal infections [30]. Plasma-derived immunoglobulins are generally highly pure for IgG. However, few IgG products exist with enriched IgM in their formulations (e.g. Pentaglobin^{™}). Delivery of plasma-derived immunoglobulins is intravenous or sub-cutaneous, ensuring systemic distribution of the immunoglobulins through the body. While Ig replacement therapy has been shown to lower pneumonia incidences in patients with immunodeficiency, it seems that they have a limited impact on upper airway infections as well as bronchial infections. Topical application of plasma-derived immunoglobulins could support a higher Ig content at the mucosal surface without having to increase systemic Ig delivery.

HRV and PA were chosen to test efficacy of plasma-derived immunoglobulins to prevent epithelial tissue infection, because of their main roles in COPD and NCFB exacerbations. To mimic better the situation in human, human primary cell-based airway model, MucilAir^{™} (Epithelix Sàrl, Geneva) was used. MucilAir^{™} is a cell model of the human airway epithelium reconstituted in vitro. MucilAir^{™}-Pool is made of a mixture of nasal or bronchial cells isolated from 14 different- or a unique donor(s) respectively. Cultured at the air-liquid interface, the model displays high trans-epithelial electrical resistance, cilia beating as well as mucus production, demonstrating the full functionality of the epithelial tissue as it would exist in vivo. Cytokine release (e.g. IL-8 and IL-6) as well as Lactate Dehydrogenase (LDH) release can be detected during infection, reflecting how infection is associated with inflammation and tissue damage in this model.

### Material and Methods

Infections of the airways starts with the deposition of pathogenic bacteria and viruses on the apical side of the airway epithelium. To reach out to the tissue, viruses will infect epithelial cells while bacteria tend to damage the cells through the secretion of exotoxins. A model of *Pseudomonas aeruginosa* infection was used to test the efficacy of plasma-derived immunoglobulins to prevent tissue damage.

### Bacterial strain

*Pseudomonas aeruginosa* (PA) used for this model is a clinical isolate obtained from the Institute of Infectious Disease (University of Bern, Switzerland)). PA is a pathogenic organism that causes disease in human and is responsible for pulmonary infections. PA were cultured on a blood agar petri dish. A colony was selected and cultured in Brain Heart Infusion (BHI) medium for 24h at 37°C and 400 revolutions per minute (RPM). On the following day, culture was diluted 1:10 with fresh BHI medium and placed for an additional hour at 37°C and 400 rpm. OD was then measured and number of bacteria was estimated from an OD/bacterial load curve, which was generated with multiple cultures prior experiment. An aliquot was collected for further dilution before and dosing, and a second aliquot was collected for further plating on blood agar plates to verify bacterial load accurately.

### Viral strains

Rhinovirus C15 is a clinical isolate (name S07-09-08-U) obtained from the Hospital of Geneva. Virus stocks were produced in MucilAir^{™} cultures and diluted in culture medium, they were not purified nor concentrated.

For the dose-response studies, rhinovirus C15 (2009) and influenza A/Switzerland/7717739/2013 (H1N1) were isolated directly on MucilAir^{™} from clinical specimen as described in [31]. Viral stocks for the experiments were produced on MucilAir^{™}, collecting apical washes with culture medium. Production of several days were pooled and quantified by qPCR, aliquoted and stored at -80 °C.

### Tissue

MucilAir^{™} (Epithelix Sàrl, Geneva) was used to mimic human bronchial tissues. For each study group, 3 MucilAir^{™} transwells were used, with each transwell originating from either one distinct donor or a mix of 14 donors used in the dose-response studies. Culture of MucilAir^{™} was performed at air-liquid interface. Medium used at the basolateral side was MucilAir^{™} culture medium (Epithelix Sàrl, Geneva), which contains growth factors and phenol red. It does not contain serum.

### Pseudomonas aeruginosa infection model and treatment

Infection model using PA is based on the deposition of as low as 10 Colony Forming Unit (CFU) of PA on the apical side of one MucilAir^{™} transwell under a volume of 10 µL. Over 24h, PA will grow to reach >10⁹ CFU/transwell. Infection leads to the release of lactate dehydrogenase (LDH)(relating to tissue damage) and pro-inflammatory molecules such as IL-8 and IL-6. Damage of the tissue is also demonstrated by the appearance of holes in the tissue and the loss of trans-epithelial electrical resistance.

In some experiments, immunoglobulins were deposited 10 minutes prior to the bacteria or simultaneously. Immunoglobulins were applied in a 10 µL final volume. The effects of immunoglobulins were compared to the vehicle solution (25 mM Proline).

### Human Rhinovirus C15 and influenza H1N1 infection model and treatment

At t=0, 15 µL of 3.8 x10⁷genome copies/mL HRV C15 (clinical strain: S07-09-08-U) stock solution in proof-of-concept experiment (Figure 10) and 10 µL of 1.0 x 10⁸ genome copies/mL for both HRV and influenza H1N1 in the dose-response studies (Figures 13 & 14) was applied on the apical side of MucilAir^{™} for 3h at 34°C and 5% CO2. Immunoglobulins were applied at the same time as viruses in 5 µL on the apical surface of MucilAir^{™} and renewed at 3.5 and 24 hours. The effects of immunoglobulins were compared to the vehicle solution (25 mM Proline). Three hours after inoculation, epithelia were washed thrice with PBS (with Ca2+/Mg2+) in order to clean the inoculum.

Cell free, apical washes (20 minutes) with 200 µL MucilAir^{™} culture media were collected at 3.5 hours post-inoculation and then 24, 48 hours and stocked at -80°C.

### Immunoglobulins

Human plasma-derived IgG preparations (IgPro10, Privigen) were prepared as reported [32]. Preparations containing IgA and IgM were obtained from an ion-exchange chromatographic side fraction used in the large-scale manufacture of IgG from human plasma. The elution fraction containing IgA and IgM was concentrated and re-buffered to 50 g/l protein in PBS by tangential-flow filtration (TFF; Pellicon XL Biomax 30, Merck Millipore). The resulting IgA/M solution, which contained IgA and IgM in a 2:1 mass ratio, was further processed to SCIgA/M, by combining in vitro IgA/M with recombinant human SC [33].

### ELISA

Pro-inflammatory cytokines release by human bronchial tissue upon infection was measured in an aliquot of the basolateral medium collected 24h post-infection. In particular, IL-8 (RnD Systems; DY208) and IL-6 (RnD Systems; DY206) were evaluated. Measurements were performed according to the user manual.

For PA ELISA, PA was cultured overnight at 37°C in BBL Todd Hewitt Broth Medium. PA were pelleted by centrifugation (3220g) for 10 minutes. Supernatant was removed and the pellet was washed twice with 0,1M carbonate buffer (pH 9,6). Pellet was resuspended in carbonate buffer and 50µl/well (4 x 10⁶ bacteria) were added onto a polysorbate plate. Coating was performed overnight at 2-8°C. The following day, wells were washed 3 times with PBS/Tween (0,05%) and blocked with PBS/FCS (2,5%) for 1.5h at room temperature. Wells were then washed 3 times with PBS/Tween (0,05%). Ig formulations (0,7 µg/ml-500 µg/ml) were added for 2h at room temperature to the wells. After washing twice with PBS/Tween (0,05%), a secondary antibody, Goat anti Human IgG/A/M-HRP (1 mg/ml, 1:2'000 in blocking buffer), was incubated for 2h at room temperature on the samples. Final washings with PBS/Tween (0,05%) were done 3 times before the TMB substrate of peroxidase was used. Blue precipitate formation is linearly proportional to the amount of enzyme in each well. Enzymatic reaction was stopped with 50 µl / well HCl 1M. Absorbance was read at 450 nm (620 nm reference wavelength). Mean blank absorbance for each triplicates was subtracted from the bacteria coated absorbance.

For the rhinovirus ELISA, a Maxisorp plate (Nunc) was coated overnight with purified rhinovirus C stock (3 x 10⁶/ml; clinical name: S07-09-09-U) (2-4°C) in 0.1M Carbonate buffer. A second Maxisorp plate was coated with 5% BSA in 0.1M Carbonate buffer, and served as "blank" plate. The following day, wells were washed 3 times with PBS/Tween (0,05%) and blocked with PBS/FCS (2,5%) for 1.5h at room temperature. Wells were then washed 3 times with PBS/Tween (0,05%). Ig formulations (0,7 µg/ml-500 µg/ml) were added for 2h at room temperature to the wells. After washing twice with PBS/Tween (0,05%), a secondary antibody, Goat anti Human IgG/A/M-HRP (1 mg/ml, 1:2'000 in blocking buffer), was incubated for 2h at room temperature on the samples. Final washings with PBS/Tween (0,05%) were done 3 times before the TMB substrate of peroxidase was used. Blue precipitate formation is linearly proportional to the amount of enzyme in each well. Enzymatic reaction was stopped with 50 µl / well HCl 1M. Absorbance was read at 450 nm (620 nm reference wavelength). Mean blank absorbance for each triplicates was subtracted from the virus coated absorbance.

### Immunohistology

Tissue damage was assessed using laser scanning confocal microscopy. Tissue were prepared as follow. MucilAir^{™} transwells were washed once in PBS and fixed overnight at 4°C in 4% paraformaldehyde. The next day, transwells were washed 3 times with PBS and tissues were permeabilized with ice cold methanol for 30 minutes at -20°C. Tissues were then washed 3 times with PBS and a blocking step was conducted overnight at 4°C using 3% Goat Serum in PBS. After another step of washes with PBS (3 times), staining was performed on the tissue for 48h-72h at 4°C with an anti-cytokeratin antibody (Abcam; ab192643)(1/200), anti-beta tubulin antibody (Abcam; ab11309)(1/200) and DAPI (Sigma D9542)(1/2000), all diluted in PBS. Tissues were then washed 3 times in PBS and transwells were separated from the tissues. Tissues were then mount onto slides, covered with mounting medium and a cover slip. The slides were kept 24h at room temperature to allow them to dry before being imaged on a Zeiss LSM800 confocal microscope.

### Transepithelial electrical resistance (TEER)

TEER is a dynamic parameter, which reflects the state of epithelia. However, it can be affected by several factors. For example, if holes were present or if tight cellular junction are lost, TEER values will reach values below 100 Ω.cm². In contrast, when epithelia is healthy, TEER value is typically above 200 Ω.cm².

Non-treated samples as well as samples treated with vehicle without virus or bacteria served as negative controls while 10% Triton X-100 was used as positive control.

To measure TEER value, 200 µL of MucilAir^{™} medium was added to the apical compartment of the MucilAir^{™} cultures, and resistance was measured with an EVOMX volt-ohm-meter (World Precision Instruments UK, Stevenage) for each condition. Resistance values (Ω) were converted to TEER (Ω.cm²) by using the following formula: $TEER \left(Ω.{cm}^{2}\right) = \left(resistance value \left(Ω\right)-100\left(Ω\right)\right) \times 0.33 \left({cm}^{2}\right)$ where 100 Ω is the resistance of the membrane and 0.33 cm² is the total surface of the epithelium.

### Lactate dehydrogenase (LDH) assay

Lactate dehydrogenase is a stable cytoplasmic enzyme that is rapidly released into the culture medium upon rupture of the plasma membrane. 100 µL basolateral medium was collected at each time-point and incubated with the reaction mixture of the Cytotoxicity Detection KitPLUS, following manufacturer's instructions (Sigma, Roche, 11644793001). The amount of the released LDH was then quantified by measuring the absorbance of each sample at 490nm with a microplate reader. Non-treated and vehicle (without virus or bacteria) served as negative control and correspond to the physiological release of LDH (≤ 5%). 10% Triton X-100 was used as a negative control and corresponds to a massive LDH release, (equal 100% cytotoxicity) To determine the percentage of cytotoxicity, the following equation was used (A = absorbance values):$Cytotoxicity \left(%\right) = \left(A \left(exp value\right)-A \left(low control\right)/A \left(high control\right)-A \left(low control\right)\right) * 100$

### Viral shedding

At each time point of the study, apical washes were conducted with 200µL MucilAir^{™} culture medium. 20 µL was further used to viral RNA extraction (QIAamp^{®} Viral RNA kit (Qiagen)), resulting in 60 µL of RNA elution volume. Viral RNA was quantified by quantitative RT-PCR (QuantiTect Probe RT-PCR, Qiagen) using 5 µL of viral RNA. Two Picornaviridae family specific, a Pan-Picornaviridae primers and Picornaviridae as well as Influenza-A specific primers and probes with FAM-TAMRA reporter-quencher dyes were also used.

Four dilutions of known concentration of HRV-A16 or H3N2 RNAs as well as control for RT-PCR were included and the plates were run on either a TaqMan ABI 7000 from Applied Biosystems or a Chromo4 PCR Detection System from Bio-Rad. Ct data were reported to the standard curve, corrected with the dilution factor and presented as genome copy number per ml on the graphs.

### Mucociliary clearance

The mucociliary clearance was monitored using a Sony XCD-U100CR camera connected to an Olympus BX51 microscope with a 5X objective. Polystyrene microbeads of 30 µm diameter (Sigma, 84135) were added on the apical surface of MucilAir^{™}. Microbead movements were video tracked at 2 frames per second for 30 images at room temperature. Three movies were taken per insert. Average beads movement velocity (µm/sec) was calculated with the ImageProPlus 6.0 software. Data are presented as mean + SEM (n=3 inserts).

### Example 1: Plasma-derived immunoglobulins interact with Pseudomonas aeruginosa

PA has been associated with many infections of the respiratory tract such as in subjects with cystic fibrosis or with severe COPD. Many different strains exist. A clinical isolate was used for its relevance to the clinical set-up. Commercially available plasma-derived immunoglobulins are mainly consisting of highly purified IgG, obtained from the fractionation of plasma pools collected from thousands of healthy adult donors. Due to its multi-donor origin, isolated immunoglobulins offer not only polyvalence and polyclonality, but also higher titers against certain pathogens as a result from vaccination. Monomeric IgA and a mix of pentameric IgM and monomeric/dimeric IgA can be isolated from the waste fraction. The inventors have previously established that polyreactive, serum-derived polymeric IgA, IgM and a mixture of the two isotypes (IgA/M) can be assembled into secretory Abs upon combination with recombinant secretory component (SC) [34]. In support of their use for local passive immunization, the molecules display high in vitro stability upon exposure to intestinal washes rich in proteases [35].

Figure 1 presents the binding of plasma-derived immunoglobulins to PA in an ELISA assay. Importantly, all plasma-derived immunoglobulins are able to bind PA clinical isolate in this assay (see material and methods section). Binding to PA was dose-dependent, with immunoglobulin amounts varying from 0.7 µg/mL to 500 µg/mL. Comparison between the immunoglobulin formulations showed differences in binding capacity of PA. For instance, mixes of IgA and IgM with or without association to SC showed the highest affinity to PA, followed by IgG and IgA.

### Example 2: Plasma-derived immunoglobulin IgG form large aggregates with PA

Immunoglobulins may account for several roles at the mucosal surfaces. They may serve as opsonins, leading to enhanced phagocytic recognition or promoting the deposition of complement and subsequent lysis. They can bind and therefore tag infected cells for destruction through a mechanism called antibody-dependent cell-mediated cytotoxicity (ADCC). Immunoglobulins can neutralize a pathogen by binding to its surface antigens and inhibiting its growth. It can also coat a pathogen and prevent its adherence to the mucosal epithelia, a mechanism called immune exclusion. At last, immunoglobulins, because of their di- or multivalent binding properties, may agglutinate microbes into larger clusters allowing for more effective recognition by the immune system and mechanical clearance by the host [36]. Secretory IgA and IgM present at the mucosal sites present 4 valences and 10 to 12 valences respectively. In contrast, IgG display only 2 valences. IgA and IgM are more prone to lead to microbe agglutination than IgG [37;38].

Figure 2 shows the analysis of immune complexes formed between IgG and PA by confocal microscopy. Using CFSE-labelled PA and Cy3-labelled plasma-derived IgG, it was surprising to detect large immune complexes of IgG-PA. Antigen binding by immunoglobulins is largely dependent on their antigen-binding (Fab) fragment. As IgG is only divalent, it is not expected to see such aggregates. This result may point out that IgG may additionally bind PA outside of the Fab region, potentially through its sugars. IgG may therefore be more potent at signaling PA to the immune system as expected.

### Example 3: Plasma-derived immunoglobulins prevent tissue damage induced by PA

PA is a pathogenic organism known for its involvement in biofilm formation as well as for its resistance to many antibiotics [39]. PA presents with many virulence factors. Some of those are exoenzymes, such as elastase A and B, Protease IV, exotoxin A, exoenzyme S or hemolysin. Exoenzymes serve at defending PA against components of the immune system as well as at participating into its toxicity and associated tissue damage.

To assess how much PA was inducing tissue damage in our infection model, we measured the release of lactate dehydrogenase (LDH), which is associated to the rupture of the plasma membrane. Experiment was run in our primary 3D cell culture system and LDH was measured in samples collected at 24h post-infection. All the immunoglobulin formulations (e.g., IgG, IgA, IgAM and slgAM) proline (vehicle) were tested. Figure 3 demonstrates that PA infection is inducing the release of LDH at a level above normal LDH level found in the medium at steady state. Importantly, when immunoglobulins were given with PA, all immunoglobulin formulations were shown to be able to prevent the release of LDH and therefore prevent tissue damage.

Another way to evaluate tissue damage is to measure trans-epithelial electrical resistance (TEER) of the tissue in vitro. Indeed, this parameter reflects the integrity of tight junction dynamics in cell culture models of an epithelial mono or multi-layer [40]. As a consequence, when tissue integrity is affected, TEER is decreased. To understand how PA infection is affecting the barrier which represents a primary epithelial tissue, TEER pre- and 24h post-infection was measured. Figure 4 shows how tissue integrity is affected by the infection and how IgG play a role in preventing it. Maximal dose of IgG and proline did not affect TEER when no bacteria is present on the apical side of the transwell. Upon PA infection, not only LDH is released as seen in Figure 3, but TEER is also decreased (proline sample). This result points out the loss of tissue integrity of the MucilAir^{™} when PA is added. To evaluate the activity of IgG in this context, increasing doses of IgG in combination with PA (dose ranging from 5 to 500 µg) were used. While the lowest IgG dose didn't show a good protection against tissue damage, increasing doses (50 to 500 µg) showed a good protection of the tissue with the best effect for the 2 highest doses.

In addition to LDH release and TEER measurements, the MucilAir^{™} tissue was viewed using microscopy to evaluate the damage occurring during PA infection. As MucilAir^{™} is a multi-layer epithelial tissue, confocal microscopy was used. The same set-up as described for Figure 4 was used. 24h post-infection, tissues were fixed and cut onto slides for staining and analysis. Figure 5 demonstrates IgG efficacy at preventing PA-induced tissue damage. Healthy tissues (controls) are represented by the sections appearing on the lower row, on the far right. Upon PA infections, large holes are appearing in the tissue (transwell treated with proline; upper row, left). Increasing doses of IgG were applied with PA (dose ranging from 5 to 500 µg). A dose-dependent effect of IgG in preventing tissue damage was observed. The lowest IgG dose did not prevent tissue damage but seemed to have an effect as holes present with a smaller surface. Increasing IgG doses were associated with no holes. However, for doses ranging from 50 to 250 µg, tissue injuries were still observable. 500 µg IgG gave the best result with a tissue looking as good as in control wells.

Altogether, all plasma-derived immunoglobulin formulations are able to prevent LDH release. Detailing the mechanism of action behind this result using IgG, it has been shown that immunoglobulin can prevent loss of tissue integrity as well as tissue damage.

### Example 4: Plasma-derived immunoglobulins prevent Pseudomonas aeruginosa-induced tissue release of pro-inflammatory cytokines

Epithelial tissues in the mucosal environment function as barriers to the external world to physically prevent microbes to enter the tissues. However, once damaged, microbes can freely enter. Therefore, to signal when there is potential infection and damage of these barriers, epithelial tissues interact with the immune system through the secretion of "danger" signals or cytokines to alert cellular components of the immune system to migrate to the tissue and offer a second layer of defense.

IL-6 and IL-8 are pro-inflammatory cytokines, which can be secreted by epithelial tissues when these tissues are insulted. In a next set of experiments, plasma-derived immunoglobulins were evaluated in the prevention of pro-inflammatory cytokines release upon PA infection. Figure 6 shows IL-8 release by MucilAir^{™} 24h post-PA infection. All the immunoglobulin formulations (e.g., IgG, IgA, IgAM and slgAM) were tested, along with proline (vehicle). Figure 6 demonstrates that IL-8 secretion is highly increased during PA infection, reaching almost a 3-fold increase. None of the immunoglobulin formulations had a significant effect on IL-8 release by the tissue at steady state. However, when applied with PA, all immunoglobulin formulations could prevent PA-induced secretion of IL-8.

To depict the effect of IgG in preventing PA-induced IL-8 release, increasing doses of IgG (dose ranging from 5 to 500 µg) were tested in combination to PA. Figure 7 detailed the dose-response of IL-8 secretion to IgG. Experiment was conducted on MucilAir^{™} generated from 3 different donors. To account for the donor-to-donor variability, IL-8 secretion post-infection was set in combination with proline as the 100% release condition. Additional conditions were calculated in relation to the 100% release. As shown in Figure 7, maximal dose of IgG and proline did not affect IL-8 release. Interestingly, IgG decreased substantially PA-induced IL-8 secretion in a dose dependent manner with the best effect obtained for the maximal dose (500 µg).

In the same way, IL-6 secretion post-PA infection was studied. Figure 8 shows IL-6 release by MucilAir^{™} 24h post-PA infection. All the immunoglobulin formulations (e.g., IgG, IgA, IgAM and slgAM) were tested, along with proline (vehicle). Figure 8 demonstrates that IL-6 secretion is highly increased by PA, reaching almost a 6-fold increase. None of the immunoglobulin formulations had a significant effect on IL-6 release by the tissue at steady state. However, when applied with PA, all immunoglobulin formulations could prevent PA-induced secretion of IL-6.

Altogether, this data set demonstrates that all immunoglobulin formulations prevent the release of pro-inflammatory cytokines such as IL-6 and IL-8 and would potentially reduce local inflammation in PA-infected subjects who received topically applied immunoglobulins as prophylaxis. Prevention of IL-8 and IL-6 secretion upon PA infection may actually translate the prevention of tissue damage by topically applied immunoglobulins against PA. Plasma-derived immunoglobulins may act via immune exclusion against PA as well as by inhibiting exoenzyme activities.

### Example 5: Plasma-derived immunoglobulins interact with human rhinoviruses

HRV are mainly known to be responsible for more than half of cold-like illness [41]. However, there are also involved in the exacerbations of chronic obstructive pulmonary disease (COPD) as well as of asthma. More than 100 serotypes exist. To assess if nebulized plasma-derived immunoglobulins could protect individuals from HRV infections, binding from a clinical isolate of HRV by different plasma-derived immunoglobulins was tested. Figure 9 shows that all immunoglobulins formulations were able to bind HRV in an ELISA assay (see material and methods section) in a dose dependent manner, with dose ranging from 0.7 µg/mL to 500 µg/mL. Binding was however different between each immunoglobulin formulation. IgG was the less potent binder while IgAM and IgA show good binding. Addition of SC to IgAM seems to decrease the potency of IgAM to bind HRV. It may point out that some of the binding is not Fab dependent.

### Example 6: Plasma-derived immunoglobulins prevent shedding and tissue damage induced by human rhinoviruses

Like other viruses, HRV are infecting cells to be able to replicate. In a following step, virions are then assembled and packaged prior to cellular export/shedding via cell lysis. Figure 10 demonstrates the effect of plasma-derived immunoglobulins to prevent HRV shedding following infection of MucilAir^{™}. When vehicle control (proline) was used, a high shedding (~10⁹ HRV C15 genome copy number/mL) of PA was detected on the apical side of the MucilAir^{™}. As a positive control, Rupintrivir, a rhinovirus 3C protease inhibitor against human rhinovirus, was used. As observed, application of Rupintrivir reduced effectively HRV shedding by 3 logs. Surprisingly, applying plasma-derived immunoglobulins at the time of infection completely reduced HRV shedding to a level which could not be detected with our assay. All immunoglobulin formulations but IgAM could show such a reduction. However, and importantly, IgAM could still decrease HRV shedding by at least 4 logs.

Plasma-derived immunoglobulins were therefore able to prevent the entrance of HRV in the epithelium and thus its subsequent replication and spreading.

While replicating, HRV can lead to cell lysis. In the context of an epithelium, we assessed if plasma-derived immunoglobulins would be able to protect epithelial cells against HRV-induced tissue damage. To evaluate this, the TEER parameter was used as a mean to assess tissue integrity post-HRV infection (Figure 11). At steady state (no infection), TEER measurement was of around 260 Ohm.cm² after treatment with vehicle (negative control). Upon HRV infection and application of the vehicle on the tissues (positive control), TEER decreased by almost 5-fold, pointing out the loss of tissue integrity following infection. As showed in Figure 11, Rupintrivir had a positive effect by preventing HRV-induced tissue damage. All plasma-derived formulations were able to prevent loss of tissue integrity when given with HRV. Immune exclusion of HRV by plasma-derived immunoglobulins proves to be sufficient to protect pulmonary tissues against PA invasion as well as PA-induced cellular damage.

### Example 7: Plasma-derived immunoglobulins reduce human rhinoviruses-induced mucociliary clearance decline

Mucociliary clearance is a key function of the bronchial tissue. Pathogens trapped into the mucus are exported out of the lungs and expectorated to prevent pathogens to stagnate onto the lungs tissues and replicate. Mucociliary clearance can be affected by different mechanisms, one of them being tissue damage.

As HRV infection is associated with tissue damage, the effect of plasma-derived immunoglobulins on mucociliary clearance 48h after HRV infection was assessed (Figure 12). At steady state (no infection), mucociliary clearance was of _{~}40mm/s after treatment with vehicle (negative control). Upon HRV infection and application of the vehicle on the tissues (positive control), mucociliary clearance decreased by 2-fold. As showed in Figure 12, Rupintrivir had a positive effect by preventing HRV-induced reduction of mucociliary clearance. All plasma-derived formulations were able to prevent the decrease of mucociliary clearance with the best effect obtain for IgA and IgM, for which no loss of mucociliary clearance was observed.

### Example 8: Dose-dependency of effects of human plasma-derived immunoglobulin formulations on human rhinovirus infection

Next it was investigated whether the observed effects were dose-dependent. Immunoglobulin formulations were added at 4, 20, 100 and 500 µg/well, and their effect on HRV expansion was assessed by measuring HRV genome copy number. Figure 13 demonstrates that plasma-derived human immunoglobulin formulations were able to inhibit HRV expansion in a dose-dependent manner.

Effect on TEER was also investigated, as described above. The immunoglobulin formulations delayed HRV-induced TEER decrease at 4 µg/well and 20 µg/well, at 100 µg/well. At 500 µg/well, the decrease was completely prevented.

Furthermore, the effect of the different doses of the immunoglobulin formulations on cilia beating frequency and on mucociliary clearance were assessed, using the same doses as specified above. Again, a dose-dependent effect was observed on cilia beating frequency and on mucociliary clearance with all immunoglobulin formulations tested.

HRV-induced IL-8 secretion on day 2 post infection was also inhibited by the plasma-derived immunoglobulin formulations; even at 4 µg/well IgAM and SlgAM achieved complete inhibition; IgG and IgA achieved a very significant reduction at 4 µg/well, and all immunoglobulin formulations achieved complete inhibition at the higher doses. HRV-induced production of RANTES was also significantly inhibited by the lowest dose of immunoglobulins used (4 µg/ml), and completely inhibited by the higher doses of all immunoglobulin formulations at day 2.

Altogether, plasma-derived immunoglobulins were able to protect in vitro pulmonary tissues against HRV infection and its associated tissue damage. Prophylactic application of plasma-derived immunoglobulins topically into the lungs of subjects at risk of pulmonary infections will give them a protection against microbes of viral or bacterial origins.

### Example 9: Effect of human plasma-derived immunoglobulin formulations on Influenza virus infection

The experiments were set up using the same protocol as for rhinovirus infection of MucilAir^{™} cultures, using Influenza strain H1N1. Oseltamivir was used as positive control at 10 µg/well. It was shown that the immunoglobulin formulations all reduced Influenza expansion in a dose-dependent manner, as shown in Figure 14.

TEER disruption by Influenza virus was also reduced by 4 µg/well and 20 µg/well of each of the immunoglobulin formulations, and completed prevented by 100 µg/ml and 500 µg/well.

An Influenza virus-induced reduction in cilia beating frequency was observed at day 4 post infection. IgG showed the best rescue effect on cilia beating frequency, showing complete restoration already with 4 µg/well. The IgA, IgAM and slgAM formulations were also able to rescue the cilia beating frequency, albeit only at 20µg/well and higher concentrations. The Ig formulations were also able to restore mucociliary clearance, reduce Influenza-induced IL-8 secretion and Influenza-induced RANTES secretion.

### Example 10: Prevention of respiratory tract infection-driven exacerbations in subjects with Chronic Obstructive Pulmonary Disease (COPD) and/or with Non-Cystic Fibrosis Bronchiectasis (NCFB) with nebulized plasma-derived immunoglobulins

Subjects with COPD and/or NCFB are subject to chronic respiratory tract infections which can participate into the exacerbation of their disease. Chronicity of these infections is driving the remodeling of their tissues, increasing the severity of disease.

As shown in the examples above, topically applied plasma-derived immunoglobulins are preventing adhesion and invasion of bacteria and viruses in primary human respiratory tract tissues in vitro. Immune exclusion of these microbes prevented tissue damage and indirectly, the release of pro-inflammatory cytokines as well as the loss of mucociliary clearance.

To break the chronicity of these infections, subjects with NCFB or mild to severe COPD, potentially in association with NCFB, are treated once or twice daily with nebulized plasma-derived immunoglobulins. Plasma-derived immunoglobulins, formulated in solution at 50 mg/mL up to 150 mg/mL, are nebulized using an active vibrating mesh nebulizer. 2-10mL of plasma-derived immunoglobulin formulation is applied in the morning and/or in the evening on a daily basis.

Reduction of infection-driven exacerbations will reduce local inflammation in COPD and NCFB subjects and will delay the progression of the disease.

### REFERENCES

[1] Vestbo et al. (2013) Am J Respir Crit Care Med 187(4):347-65.
[2] Flume et al. (2018) Lancet 392(10150):880-90.
[3] Chalmers et al. (2018) Am J Respir Crit Care Med 197(11):1410-20.
[4] Global strategy for the diagnosis, management, and prevention of chronic obstructive pulmonary disease, 2018 report; Global initiative for chronic obstructive lung disease (GOLD).
[5] Mohan A et al. (2010) Respirology 15(3):536-42.
[6] Molyneaux et al. (2013) Am J Respir Crit Care Med 188(10):1224-31.
[7] Hassett et al. (2014) J Microbiol 52(3):211-26.
[8] Miravitlles et al. (2017) Respiratory Research 18:198.
[9] Singanayagam et al. (2018) Nature Communications 9(1):2229.
[10] British Thoracic Society: Guideline for non-CF Bronchiectasis (2010) Thorax vol. 65, Supp. 1.
[11] Spirometry for health care providers (June 2010). Published by the Global Initiative for Chronic Obstructive Lung Disease (GOLD).
[12] Published by the Association for Respiratory Technology and Physiology (www.artp.org.uk).
[13] Agarwal and Cunningham-Rundles (2007) Ann Allergy Asthma Immunol. 99(3):281-3.
[14] Normansell (1982) CRC critical reviews in clinical laboratory sciences 17(2):103-66.
[15] Hill et al. (1998) Thorax 53:463-8.
[16] Stucki et al. (2008) Biologicals 36(4):239-47.
[17] WO/2012/030664.
[18] Gennaro (2000) Remington: The Science and Practice of Pharmacy. 20th edition, ISBN: 0683306472.
[19] Donaldson and Wedzicha (2014) Int J of COPD 9:1101-10.
[20] Chairatana and Nolan (2016) Crit. Rev. Biochem. Mol. Biol. 113:1-12.
[21] Mantis et al. (2011) Mucosal Immunol. 4:603-11.
[22] Corthésy (2013) Autoimmun. Rev. 12:661-5.
[23] Strugnell and Wijburg (2011) Nat. Rev. Microbiol. 8:656-67.
[24] Mathias et al. (2013) Infect. Immun. 81:3027-34.
[25] Levinson et al. (2015) Infect. Immun. 83:1674-83.
[26] Brandtzaeg (2009) Scand. J. Immunol. 70:505-15.
[27] Brandtzaeg (2003) Vaccine 21:3382-88.
[28] Corthésy (2003) Curr. Pharm. Biotechnol. 4:51-67.
[29] Corthésy (2002) Trends Biotechnol. 20:65-71.
[30] Lucas et al. (2010) J Allergy Clin Immunol. 125(6):1354-60.
[31] Essaidi-Laziosi et al. (2018) J. Allergy Clin Immunol. 141(6):2074-2084
[32] Cramer et al. (2009) Vox Sang. 96:219-25.
[33] Phalipon et al. (2002) Immunity 17:107-15.
[34] Longet et al. (2013) J. Biol. Chem. 288:4085-94.
[35] Crottet and Corthésy (1998) J. Immunol. 161:5445-53.
[36] Roche et al. (2015) mucosal immunology 8(1):176-85.
[37] Bioley et al. (2017) Front Immunol. 8:1043.
[38] Longet et al. (2014) JBC 289(31):21617-26.
[39] Azham et al. (2018) Drug Discov Today S1359-6446(18):30235-6.
[40] Srinivasan et al. (2015) J Lab Autom 20(2):107-26.
[41] Jacobs et al. (2013) Clinical Microbiology Reviews 26(1):135-62.

## Claims

1. A composition comprising human plasma-derived IgG for use in the prevention or treatment of an acute exacerbation in a human subject with a chronic lung disease, wherein the composition is administered to the respiratory tract of the subject, wherein the chronic lung disease is chronic obstructive pulmonary disease (COPD) or non-cystic fibrosis bronchiectasis (NCFB), and wherein the acute exacerbation is caused by a viral or bacterial infection of the respiratory tract, wherein the subject has:
(a) a rhinovirus respiratory tract infection, and/or
(b) a *Pseudomonas aeruginosa* respiratory tract infection.

2. The composition for use according to claim 1, wherein the COPD is moderate to severe COPD.

3. The composition for use according to any preceding claim, wherein the subject has a plasma IgG level of less than 700 mg/dl.

4. The composition for use according to any preceding claim, wherein the subject has experienced one or more acute exacerbations in the 12 months prior to prevention or treatment starting.

5. The composition for use according to any preceding claim, wherein the subject has one or more detectable pro-inflammatory cytokines, such as IL-1β and/or IL-6 and/or IL-8, in his or her sputum.

6. The composition for use according to any preceding claim, wherein the subject suffers from pneumonia.

7. The composition for use according to any preceding claim, wherein the IgG reduces inflammation in the respiratory tract of the subject.

8. The composition for use according to any preceding claim, wherein the IgG reduces the level of one or more pro-inflammatory cytokines in the respiratory tract of the subject, for example IL-1β and/or IL-6 and/or IL-8.

9. The composition for use according to any preceding claim, wherein the IgG causes immune exclusion of at least one potentially pathogenic microbe infecting the respiratory tract of the subject.

10. The composition for use according to any preceding claim, wherein the IgG reduces direct damage to epithelial tissue in the subject caused by at least one pathogen, for example the polyclonal immunoglobulin reduces the activity of exoenzymes, reduces loss of epithelial barrier integrity and/or reduces viral shedding.

11. The composition for use according to any preceding claim, wherein the composition is at least 95%, more particularly at least 98% IgG.

12. The composition for use according to claim 11, wherein the composition comprises proline, for example from about 210 to about 290 mmol/I of L proline, preferably about 250 mmol/I of L proline.

13. The composition for use according to any preceding claim, wherein the composition is administered as an aerosol.

14. The composition for use according to any preceding claim, wherein the composition is an aqueous solution having a polyclonal immunoglobulin concentration of 50 mg/ml to 150 mg/ml, for example about 100 mg/ml.

15. The composition for use according to any preceding claim, wherein the composition is administered in 2-10 ml.

16. The composition for use according to any preceding claim, wherein the composition is administered once every 48 hours or once every 24 hours or once every 12 hours during the treatment.

17. The composition for use according to any preceding claim, wherein the composition is administered during the fall and winter months.

18. The composition for use according to any preceding claim, wherein the composition is administered in a combination therapy with one or more of an antibiotic, a corticosteroid, a beta2 agonist and an anticholinergic bronchodilator.

19. The composition for use according to claim 13, wherein the composition is a dry powder.

## Patentansprüche

1. Zusammensetzung, die aus menschlichem Plasma stammendes IgG umfasst, zur Verwendung bei der Verhütung oder Behandlung einer akuten Exazerbation bei einem menschlichen Individuum mit einer chronischen Lungenerkrankung, wobei die Zusammensetzung an die Atemwege des Individuums verabreicht wird, wobei es sich bei der chronischen Lungenerkrankung um eine chronische obstruktive Lungenerkrankung (COPD) oder eine nicht-zystische-Fibrose-Bronchiektasie (NCFB) handelt und wobei die akute Exazerbation durch eine virale oder bakterielle Infektion der Atemwege verursacht wird, wobei das Individuum Folgendes aufweist:
(a) eine Rhinovirus-Infektion der Atemwege und/oder
(b) eine *Pseudomonas-aeruginosa*-Infektion der Atemwege.

2. Zusammensetzung zur Verwendung gemäß Anspruch 1, wobei es sich bei der COPD um mittelschwere bis schwere COPD handelt.

3. Zusammensetzung zur Verwendung gemäß einem beliebigen vorhergehenden Anspruch, wobei das Individuum einen IgG-Plasmaspiegel von weniger als 700 mg/dl aufweist.

4. Zusammensetzung zur Verwendung gemäß einem beliebigen vorhergehenden Anspruch, wobei das Individuum in den 12 Monaten vor Beginn der Verhütung oder Behandlung eine oder mehrere akute Exazerbationen erfahren hat.

5. Zusammensetzung zur Verwendung gemäß einem beliebigen vorhergehenden Anspruch, wobei das Individuum ein oder mehrere nachweisbare entzündungsfördernde Zytokine, wie etwa IL-1β und/oder IL-6 und/oder IL-8, in seinem Sputum aufweist.

6. Zusammensetzung zur Verwendung gemäß einem beliebigen vorhergehenden Anspruch, wobei das Individuum an Lungenentzündung leidet.

7. Zusammensetzung zur Verwendung gemäß einem beliebigen vorhergehenden Anspruch, wobei das IgG eine Entzündung in den Atemwegen des Individuums verringert.

8. Zusammensetzung zur Verwendung gemäß einem beliebigen vorhergehenden Anspruch, wobei das IgG den Spiegel eines oder mehrerer entzündungsfördernder Zytokine, zum Beispiel IL-1β und/oder IL-6 und/oder IL-8, in den Atemwegen des Individuums senkt.

9. Zusammensetzung zur Verwendung gemäß einem beliebigen vorhergehenden Anspruch, wobei das IgG eine Immunexklusion mindestens eines potentiell pathogenen Mikroorganismus bewirkt, mit welchem die Atemwege des Individuums infiziert sind.

10. Zusammensetzung zur Verwendung gemäß einem beliebigen vorhergehenden Anspruch, wobei das IgG eine direkte Schädigung des Epithelgewebes bei dem Individuum verringert, wie sie durch mindestens ein Pathogen verursacht wird, wobei das polyklonale Immunglobulin beispielsweise die Aktivität von Exoenzymen verringert, einen Integritätsverlust der Epithelbarriere verringert und/oder die Virusausscheidung verringert.

11. Zusammensetzung zur Verwendung gemäß einem beliebigen vorhergehenden Anspruch, wobei die Zusammensetzung zu mindestens 95 %, insbesondere mindestens 98 %, aus IgG besteht.

12. Zusammensetzung zur Verwendung gemäß Anspruch 11, wobei die Zusammensetzung Prolin umfasst, zum Beispiel ungefähr 210 bis ungefähr 290 mmol/l L an Prolin, vorzugsweise ungefähr 250 mmol/l L an Prolin.

13. Zusammensetzung zur Verwendung gemäß einem beliebigen vorhergehenden Anspruch, wobei die Zusammensetzung als Aerosol verabreicht wird.

14. Zusammensetzung zur Verwendung gemäß einem beliebigen vorhergehenden Anspruch, wobei es sich bei der Zusammensetzung um eine wässrige Lösung mit einer Konzentration an polyklonalem Immunglobulin von 50 mg/ml bis 150 mg/ml, zum Beispiel ungefähr 100 mg/ml, handelt.

15. Zusammensetzung zur Verwendung gemäß einem beliebigen vorhergehenden Anspruch, wobei die Zusammensetzung in 2 bis 10 ml verabreicht wird.

16. Zusammensetzung zur Verwendung gemäß einem beliebigen vorhergehenden Anspruch, wobei die Zusammensetzung während der Behandlung einmal alle 48 Stunden oder einmal alle 24 Stunden oder einmal alle 12 Stunden verabreicht wird.

17. Zusammensetzung zur Verwendung gemäß einem beliebigen vorhergehenden Anspruch, wobei die Zusammensetzung während der Herbst- und Wintermonate verabreicht wird.

18. Zusammensetzung zur Verwendung gemäß einem beliebigen vorhergehenden Anspruch, wobei die Zusammensetzung in einer Kombinationstherapie mit einem oder mehreren von einem Antibiotikum, einem Kortikosteroid, einem beta2-Agonisten und einem anticholinergen Bronchodilatator verabreicht wird.

19. Zusammensetzung zur Verwendung gemäß Anspruch 13, wobei die Zusammensetzung ein trockenes Pulver ist.

## Revendications

1. Composition comprenant des IgG issues de plasma humain pour une utilisation dans la prévention ou le traitement d'une exacerbation aiguë chez un sujet humain atteint de maladie pulmonaire chronique, la composition étant administrée aux voies respiratoires du sujet, la maladie pulmonaire chronique étant la bronchopneumopathie chronique obstructive (BPCO) ou la bronchectasie non liée à la mucoviscidose (BNLM), et l'exacerbation aiguë étant provoquée par une infection virale ou bactérienne des voies respiratoires, le sujet présentant :
(a) une infection des voies respiratoires à rhinovirus, et/ou
(b) une infection des voies respiratoires à *Pseudomonas aeruginosa*.

2. Composition selon la revendication 1, la BPCO étant une BPCO modérée à sévère.

3. Composition selon l'une quelconque des revendications précédentes, le sujet présentant un taux plasmatique d'IgG inférieur à 700 mg/dL.

4. Composition selon l'une quelconque des revendications précédentes, le sujet ayant subi une ou plusieurs exacerbations aiguës dans les 12 mois précédant le début de la prévention ou du traitement.

5. Composition selon l'une quelconque des revendications précédentes, le sujet présentant une ou plusieurs cytokines pro-inflammatoires détectables, telles que IL-1β et/ou IL-6 et/ou IL-8, dans ses expectorations.

6. Composition selon l'une quelconque des revendications précédentes, le sujet souffrant d'une pneumonie.

7. Composition selon l'une quelconque des revendications précédentes, les IgG réduisant l'inflammation dans les voies respiratoires du sujet.

8. Composition selon l'une quelconque des revendications précédentes, les IgG réduisant le taux d'une ou plusieurs cytokines pro-inflammatoires dans les voies respiratoires du sujet, par exemple IL-1β et/ou IL-6 et/ou IL-8.

9. Composition selon l'une quelconque des revendications précédentes, les IgG provoquant l'exclusion immunitaire d'au moins un microbe potentiellement pathogène infectant les voies respiratoires du sujet.

10. Composition selon l'une quelconque des revendications précédentes, les IgG réduisant les dommages directs causés au tissu épithélial chez le sujet par au moins un agent pathogène, par exemple l'immunoglobuline polyclonale réduit l'activité des exoenzymes, réduit la perte d'intégrité de la barrière épithéliale et/ou réduit l'excrétion virale.

11. Composition selon l'une quelconque des revendications précédentes, la composition étant constituée d'au moins 95 %, plus particulièrement d'au moins 98 % d'IgG.

12. Composition selon la revendication 11, la composition comprenant de la proline, par exemple d'environ 210 à environ 290 mmol/L de L-proline, de préférence environ 250 mmol/L de L-proline.

13. Composition selon l'une quelconque des revendications précédentes, la composition étant administrée sous forme d'aérosol.

14. Composition selon l'une quelconque des revendications précédentes, la composition étant une solution aqueuse ayant une concentration en immunoglobuline polyclonale de 50 mg/mL à 150 mg/mL, par exemple environ 100 mg/mL.

15. Composition selon l'une quelconque des revendications précédentes, la composition étant administrée dans 2 à 10 mL.

16. Composition selon l'une quelconque des revendications précédentes, la composition étant administrée toutes les 48 heures ou toutes les 24 heures ou toutes les 12 heures pendant le traitement.

17. Composition selon l'une quelconque des revendications précédentes, la composition étant administrée pendant les mois d'automne et d'hiver.

18. Composition selon l'une quelconque des revendications précédentes, la composition étant administrée dans une polythérapie avec un ou plusieurs agents parmi un antibiotique, un corticostéroïde, un agoniste bêta2 et un bronchodilatateur anticholinergique.

19. Composition selon la revendication 13, la composition étant une poudre sèche.
